(19) Europäisches Patentamt
European Patent Office
Office européen des brevets

(11) Veröffentlichungsnummer: **0 043 446**
**B1**

(12) # EUROPÄISCHE PATENTSCHRIFT

(45) Veröffentlichungstag der Patentschrift: **22.02.84**

(51) Int. Cl.³: **C 07 C 149/26,** C 07 C 177/00, C 07 D 309/12, C 07 D 307/93

(21) Anmeldenummer: **81104261.3**

(22) Anmeldetag: **03.06.81**

(54) **Neue 13-Thiaprostaglandin-Zwischenprodukte, Verfahren zu ihrer Herstellung und ihre Verwendung zur Herstellung von 13-Thiaprostaglandin-Derivaten.**

(30) Priorität: **04.07.80 DE 3025325**

(43) Veröffentlichungstag der Anmeldung: **13.01.82 Patentblatt 82/2**

(45) Bekanntmachung des Hinweises auf die Patenterteilung: **22.02.84 Patentblatt 84/8**

(84) Benannte Vertragsstaaten: **AT BE CH DE FR GB IT LI NL SE**

(56) Entgegenhaltungen: **GB - A - 2 013 661**

(73) Patentinhaber: **Merck Patent Gesellschaft mit beschränkter Haftung, Frankfurter Strasse 250, D-6100 Darmstadt (DE)**

(72) Erfinder: **Riefling, Bernhard, Dr., Waldstrasse 27, D-6104 Seeheim (DE)**
Erfinder: **Radunz, Hans-Eckart, Dr., Am Klingenteich 5, D-6109 Mühltal 4 (DE)**

EP 0 043 446 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Neue 13-Thiaprostaglandin-Zwischenprodukte, Verfahren zu ihrer Herstellung und ihre Verwendung zur Herstellung von 13-Thiaprostaglandin-Derivaten

Die Erfindung betrifft neue 13-Thiaprostaglandin-Zwischenprodukte, die für die stereospezifische Herstellung von 13-Thiaprostaglandin-Derivaten äußerst wertvoll sind, ihre Herstellung und ihre Verwendung zur Herstellung von bekannten 13-Thiaprostaglandin-Derivaten. Außerdem betrifft die Erfindung ein neues Verfahren zur Herstellung von bekannten 13-Thiaprostaglandin-Derivaten, ausgehend von den neuen Zwischenprodukten.

13-Thiaprostaglandin-Derivate sind aus der DE-OS 2 256 537, der DE-OS 2 422 924 und der DE-OS 2 644 972 bekannt und besitzen wertvolle pharmakologische Eigenschaften. So treten vor allem bei den 13-Thiaprostaglandin-Derivaten vom E-Typ blutdrucksenkende Wirkungen auf, die sich z. B. an der barbituratnarkotisierten Katze bei Dauerinfusion zeigen. In diesem Test wird der arterielle Blutdruck kymographisch registriert. Die Testsubstanzen werden über einen Zeitraum von 10 Minuten in wässeriger Propylenglykol-Lösung infundiert.

Außerdem treten bei den 13-Thiaprostaglandin-Derivaten vasodilatorische, antiphlogistische, diuretische, Bronchien entkrampfende, die Magensaftsekretion, die Thrombozyten-Aggregation, den Lipidabbau und die Noradrenalin-Freisetzung hemmende sowie nasenschleimhautabschwellende Eigenschaften auf, die ebenfalls nach hierfür geläufigen Methoden ermittelt werden können.

Die 13-Thiaprostaglandin-Derivate können auch die Funktion des corpus luteum, den Eitransport durch den Eileiter, die Nidation und die Fertilität beeinflussen. So zeigen insbesondere die 13-Thiaprostaglandin-Derivate vom F-Typ eine oestrus-synchronisierende Wirkung, beispielsweise bei Rindern, Pferden, Schafen, Schweinen und Hunden. Um diese Wirkung auszunutzen, wird der Wirkstoff intramuskulär injiziert, vorteilhaft zwischen dem 7. Tag und dem 12. Tag des Zyklus.

Mehrere Verfahren zur Herstellung dieser 13-Thiaprostaglandin-Derivate sind bekannt, haben jedoch gewisse Nachteile. Die Ausbeuten sind nicht immer befriedigend, die Synthesen laufen über eine Vielzahl von Reaktionsstufen, die Aufarbeitung ist schwierig, und die Reinheit der erhaltenen Produkte läßt zu wünschen übrig. Insbesondere besteht bei der infolge ihrer komplexen Stereochemie schwierigen Synthese dieser 13-Thiaprostaglandin-Derivate ein Bedürfnis nach Verfahren, die in wenigen Reaktionsschritten zu isomeren- und epimerenreinen Produkten führen.

Der Erfindung lag deshalb die Aufgabe zugrunde, neue Verbindungen aufzufinden, die infolge ihrer Regiospezifität und Stabilität als Ausgangsmaterialien für die Herstellung von 13-Thiaprostaglandin-Derivaten geeignet sind. Weiterhin bestand die Aufgabe, ausgehend von diesen neuen Verbindungen, ein neues Verfahren zur Herstellung von 13-Thiaprostaglandin-Derivaten aufzufinden, das die Nachteile der bekannten Verfahren nicht oder nur in geringerem Maße besitzt. Diese Aufgabe wird gelöst durch die Bereitstellung der neuen 13-Thiaprostaglandin-Zwischenprodukte.

Gegenstand der Erfindung sind somit Verbindungen der allgemeinen Formel I

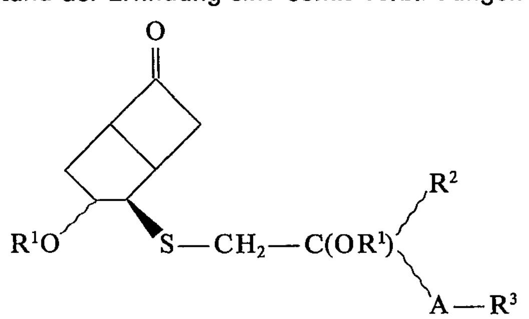

(I)

worin

A eine C—C-Einfachbindung, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ oder $-CH_2O-$,

$R^1$ Wasserstoff oder eine solvolytisch oder hydrogenolytisch abspaltbare Schutzgruppe,

$R^2$ H oder Alkyl mit 1 bis 3 C-Atomen,

$R^3$ Alkyl mit 3 bis 5 C-Atomen; Phenyl oder ein- bis dreifach durch F, Cl, OH, $OCH_3$, $OC_2H_5$, $CF_3$ oder Alkyl mit 1 bis 3 C-Atomen substituiertes Phenyl; oder, wenn A nicht $-CH_2O-$ ist, zusätzlich auch Pyridyl; Thienyl; Naphthyl oder Alkoxy mit 1 bis 4 C-Atomen bedeuten,

▬ eine *β*-ständige Bindung anzeigt und eine Wellenlinie (~~) bedeutet, daß diese Bindungen *α*- oder *β*-ständig sein können.

Die Verbindungen der Formel I enthalten 4 asymmetrische C-Atome am Fünfring. In der Thioäther-Seitenkette können weitere Asymmetriezentren auftreten.

Die Verbindungen der Formel I können daher in einer Vielzahl stereoisomerer Formen auftreten.

Gegenstand der Erfindung sind neben den einzelnen Racematen und racemischen Gemischen auch die optisch aktiven Isomeren der Formel I.

Gegenstand der Erfindung ist ferner ein Verfahren zur Herstellung einer Verbindung der Formel I, das dadurch gekennzeichnet ist, daß man das 2-Brom-3-hydroxy-bicyclo(3.2.0)heptan-6-on mit einem Thiol oder Thiolat der allgemeinen Formel II

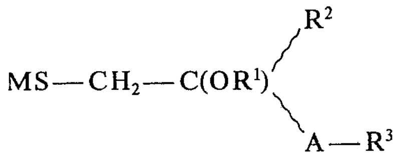

(II)

worin

M, H ein Äquivalent eines Metallatoms oder Ammonium bedeutet und
A und $R^1$ bis $R^3$ die oben angegebenen Bedeutungen haben.

umsetzt
und daß man gegebenenfalls eine oder mehrere vorhandene Hydroxygruppe(n) durch eine solvolytisch oder hydrogenolytisch abspaltbare Schutzgruppe blockiert.

Ebenso ist Gegenstand der Erfindung die Verwendung der Verbindungen der allgemeinen Formel I zur Herstellung von 13-Thiaprostaglandin-Derivaten der allgemeinen Formel III

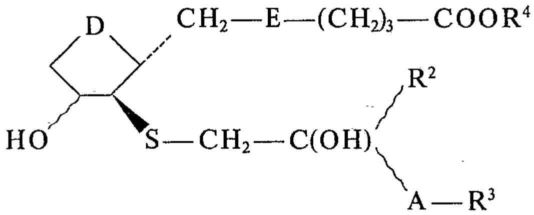

(III)

worin

D $-CO-$ oder $-CHOH-$,
E $-CH_2-CH_2-$ oder $-CH=CH-$,
$R^4$ H, Alkyl mit 1 bis 4 C-Atomen oder $-Q-NH-COR^5$,
Q 1,4-Phenylen oder 1,4-Naphthylen und
$R^5$ $NH_2$, $CH_3$, Phenyl, p-Acetylaminophenyl, p-Benzoylaminophenyl oder Phenylamino bedeuten,

A, $R^2$ und $R^3$ die oben angegebenen Bedeutungen haben,
... eine $\alpha$-ständige Bindung und ◀ eine $\beta$-ständige Bindung anzeigt und eine Wellenlinie (~~~) bedeutet, daß diese Bindungen $\alpha$- oder $\beta$-ständig sein können,

sowie ihrer physiologisch unbedenklichen Salze, wenn $R^4$ Wasserstoff bedeutet,
die dadurch gekennzeichnet ist, daß man eine Verbindung der allgemeinen Formel I ultraviolett bestrahlt und daß man das erhaltene Lactol der allgemeinen Formel IV

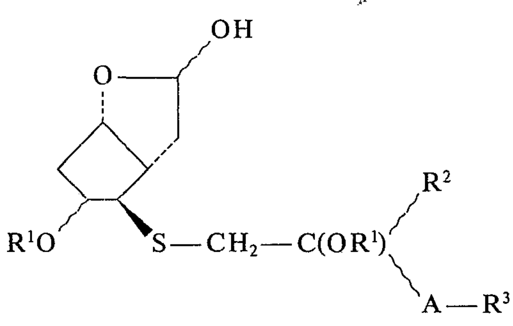

(IV)

worin

A und $R^1$ bis $R^3$ die oben angegebenen Bedeutungen haben,
... eine $\alpha$-ständige Bindung und ◀ eine $\beta$-ständige Bindung anzeigt und eine Wellenlinie (~~~) bedeutet, daß diese Bindungen $\alpha$- oder $\beta$-ständig sein können,

mit einer Verbindung der allgemeinen Formel V

$$[(R^6)_3P^{(+)}-CH_2)_4-COOR^4]X^{(-)} \qquad V$$

worin

$R^6$ Alkyl mit 1–4 C-Atomen oder Phenyl,
X Cl, Br oder J bedeutet und
$R^4$ die oben angegebene Bedeutung hat,

umsetzt,
und daß man gegebenenfalls eine Verbindung der Formel III mit E = –CH=CH– durch Hydrierung in eine Verbindung der Formel III mit E = $-CH_2-CH_2-$ überführt und/oder vorhandene Schutzgruppen in $C_{11}$- und $C_{15}$-Stellung solvolytisch oder hydrogenolytisch abspaltet und/oder daß man zur Herstellung der 13-Thiaprostaglandin-E-Analogen der allgemeinen Formel III auf einer beliebigen Stufe die Hydroxygruppen in $C_{11}$- und $C_{15}$-Stellung selektiv durch solvolytisch oder hydrogenolytisch abspaltbare Schutzgruppen blockiert, die freie Hydroxygruppe in $C_9$-Stellung zur Ketogruppe oxidiert und die Schutzgruppen in $C_{11}$- und $C_{15}$-Stellung solvolytisch oder hydrogenolytisch abspaltet,
und/oder daß man gegebenenfalls eine Verbindung der Formel III mit $R^4$ = H durch Umsetzen mit einem entsprechenden veresternden Mittel in eine andere Verbindung der Formel III mit $R^4$ = Alkyl mit 1 bis 4 C-Atomen umwandelt,
und/oder daß man gegebenenfalls eine Verbindung der Formel III mit $R^4$ = H durch Umsetzen mit einer Verbindung der Formel VI

$$HO-Q-NH-COR^5 \qquad VI$$

in eine andere Verbindung der Formel III mit $R^4$ = $-Q-NH-COR^5$ umwandelt,
und/oder daß man gegebenenfalls eine Verbindung der Formel III mit $R^4$ = Alkyl mit 1–4 C-Atomen oder $-Q-NH-COR^5$ durch Umsetzen mit einem solvolysierenden Mittel in eine andere Verbindung der Formel III mit $R^4$ = H umwandelt,
und/oder daß man gegebenenfalls eine Verbindung der Formel III in ihre Racemate und/oder Enantiomeren aufspaltet,
und/oder daß man gegebenenfalls eine Verbindung der Formel III mit $R^4$ = H durch Behandeln mit einer Base in eines ihrer physiologisch unbedenklichen Salze umwandelt oder aus einem ihrer Salze durch Behandeln mit einer Säure in Freiheit setzt.

Weiterhin ist Gegenstand der Erfindung ein Verfahren zur Herstellung von 13-Thiaprostaglandin-Derivaten der allgemeinen Formel III

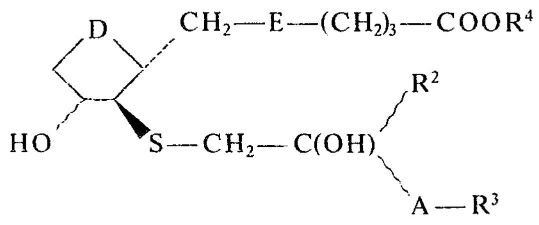

(III)

worin

D –CO– oder –CHOH–,
E $-CH_2-CH_2-$ oder –CH=CH–,
$R^4$ H, Alkyl mit 1 bis 4 C-Atomen oder $-Q-NH-COR^5$,
Q 1,4-Phenylen oder 1,4-Naphthylen und
$R^5$ $NH_2$, $CH_3$, Phenyl, p-Acetylaminophenyl, p-Benzoylaminophenyl oder Phenylamino bedeuten,
A, $R^2$ und $R^3$ die oben angegebenen Bedeutungen haben,
... eine $\alpha$-ständige Bindung und ◄ eine $\beta$-ständige Bindung anzeigt und eine Wellenlinie (~~) bedeutet, daß diese Bindungen $\alpha$- oder $\beta$-ständig sein können,

sowie ihrer physiologisch unbedenklichen Salze, wenn $R^4$ Wasserstoff bedeutet,
das dadurch gekennzeichnet ist, daß man eine Verbindung der allgemeinen Formel I ultraviolett bestrahlt und daß man das erhaltene Lactol der allgemeinen Formel IV

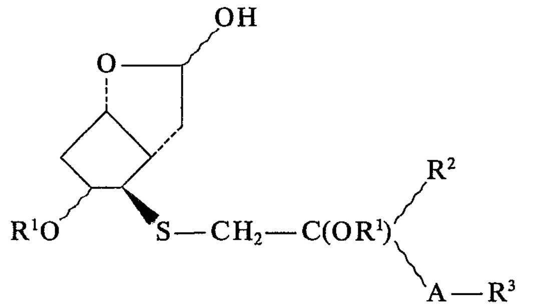

(IV)

worin

A und $R^1$ bis $R^3$ die oben angegebenen Bedeutungen haben,
... eine $\alpha$-ständige Bindung und ◄ eine $\beta$-ständige Bindung anzeigt und eine Wellenlinie (~~) bedeutet, daß diese Bindungen $\alpha$- oder $\beta$-ständig sein können,

mit einer Verbindung der allgemeinen Formel V

$$[(R^6)_3P^{(+)}-CH_2)_4-COOR^4]\,X^{(-)} \qquad V$$

worin

$R^6$ Alkyl mit 1—4 C-Atomen oder Phenyl,
X Cl, Br oder J bedeutet und
$R^4$ die oben angegebene Bedeutung hat,

umsetzt,
und daß man gegebenenfalls eine Verbindung der Formel III mit E = —CH=CH— durch Hydrierung in eine Verbindung der Formel III mit E = $-CH_2-CH_2-$ überführt und/oder vorhandene Schutzgruppen in $C_{11}$- und $C_{15}$-Stellung solvolytisch oder hydrogenolytisch abspaltet und/oder daß man zur Herstellung der 13-Thiaprostaglandin-E-Analogen der allgemeinen Formel III auf einer beliebigen Stufe die Hydroxygruppen in $C_{11}$- und $C_{15}$-Stellung selektiv durch solvolytisch oder hydrogenolytisch abspaltbare Schutzgruppen blockiert, die freie Hydroxygruppe in $C_9$-Stellung zur Ketogruppe oxidiert und die Schutzgruppen in $C_{11}$- und $C_{15}$-Stellung solvolytisch oder hydrogenolytisch abspaltet,
und/oder daß man gegebenenfalls eine Verbindung der Formel III mit $R^4$ = H durch Umsetzen mit einem entsprechenden veresternden Mittel in eine andere Verbindung der Formel III mit $R^4$ = Alkyl mit 1 bis 4 C-Atomen umwandelt,
und/oder daß man gegebenenfalls eine Verbindung der Formel III mit $R^4$ = H durch Umsetzen mit einer Verbindung der Formel VI

$$HO-Q-NH-COR^5 \qquad VI$$

in eine andere Verbindung der Formel III mit $R^4$ = $-Q-NH-COR^5$ umwandelt,
und/oder daß man gegebenenfalls eine Verbindung der Formel III mit $R^4$ = Alkyl mit 1—4 C-Atomen oder $-Q-NH-COR^5$ durch Umsetzen mit einem solvolysierenden Mittel in eine andere Verbindung der Formel III mit $R^4$ = H umwandelt,
und/oder daß man gegebenenfalls eine Verbindung der Formel III in ihre Racemate und/oder Enantiomeren aufspaltet,
und/oder daß man gegebenenfalls eine Verbindung der Formel III mit $R^4$ = H durch Behandeln mit einer Base in eines ihrer physiologisch unbedenklichen Salze umwandelt oder aus einem ihrer Salze durch Behandeln mit einer Säure in Freiheit setzt.

Die Verbindungen der Formel I sind wertvolle Zwischenprodukte bei der stereospezifischen Synthese von 13-Thiaprostaglandin-Derivaten. Da die das Schwefelatom enthaltende Seitenkette bereits auf einer frühen Stufe der Synthese eingeführt wird, ist es möglich, störende Isomere und Epimere bereits im Anfangsstadium abzutrennen. Als Folge davon fällt in der letzten Stufe das gewünschte 13-Thiaprostaglandin-Derivat isomeren- und epimerenrein an. Auch muß es als überraschend und nicht vorhersehbar angesehen werden, daß die Umsetzung eines Thiols der Formel II mit dem 2-Brom-3-hydroxy-bicyclo-(3.2.0)-heptan-6-on in einer äußerst regiospezifischen Reaktion fast ausschließlich zu den gewünschten Verbindungen der Formel I führt. Das unerwünschte Isomere entsteht nur in Spuren und läßt sich außerdem leicht durch Umkristallisieren abtrennen.

Eine ähnliche, regiospezifische Reaktion — die Umsetzung von 2,3-Epoxy-bicyclo(3.2.0)heptan-6-on bzw. Derivaten dieser Verbindung mit einem 1 bis 12 C-Atome enthaltenden organometallischen Reagens — ist zwar aus der DE-OS 2 800 929 bekannt; es gab jedoch keinerlei Hinweise, daß die hier vorliegende Umsetzung des 2-Brom-3-hydroxy-bicyclo(3.2.0)heptan-6-ons mit einem Thiol bzw.

einem Thiolat in dieser äußerst regiospezifischen Weise zu den gewünschten Verbindungen führt.

Darüber hinaus läßt sich die erfindungsgemäße Reaktion in einfachster Weise bei Raumtemperatur durchführen, während die in der DE-OS 2 800 929 beschriebene Raktion die Einhaltung spezieller Verfahrensmaßnahmen erfordert.

Auch das neue Verfahren zur Herstellung der 13-Thiaprostaglandin-Derivate der Formel III, das von den neuen Verbindungen der Formel I ausgeht, besitzt gegenüber den bisherigen Verfahren entscheidende Vorteile.

Die Ultraviolett-Bestrahlung einer Verbindung der Formel I führt unter milden Bedingungen bei Raumtemperatur und unter Vermeidung von gefährlichen und teuren Reagentien zu den Lactolen der Formel IV. Das bisherige Verfahren dagegen (vgl. Corey et al., Tetrahedron Letters, 311, 1970) enthält die Reduktion eines Lactons, ein Syntheseschritt, bei dem das teure und gefährliche Diisobutylaluminiumhydrid bei tiefer Temperatur zum Einsatz kommt. Die Umsetzung von Cyclobutanonen zu cyclischen Acetalen durch Ultraviolett-Bestrahlung in alkoholischen Lösungen wurde bereits eingehend untersucht (Yates et al., Tetrahedron Letters, 1964, 1739; Yates et al., Accounts Chem. Res. 1975, 8, 209; Turro et al., Adv. Photochem., 1974, 9, 197). Auch die Umsetzung des 2,3-Epoxy-bicyclo(3.2.0)-heptan-6-on zu 2-Oxa-3-methoxy-6,7-epoxy-bicyclo(3.3.0)octan durch UV-Bestrahlung ist bereits bekannt (Crossland et al., J. C. S., Chem. Comm. 1978, 661). Es muß jedoch als überraschend angesehen werden, daß diese Reaktion auch bei den vorliegenden Verbindungen, die eine Hydroxyalkylthio-Seitenkette enthalten, in so hervorragender Weise durchgeführt werden kann.

Die anschließende Umsetzung einer Verbindung der Formel IV mit einem Wittig-Reagens der Formel V zu den 13-Thiaprostaglandin-F-Analogen der Formel III ist bereits in der DE-OS 25 50 004 beschrieben.

Die Verbindungen der Formel III sind strukturell mit den Prostaglandinen verwandt, die sich von der 7-(2-Octylcyclopentyl)-heptansäure (Prostansäure) ableiten. Daher können die Verbindungen der Formel III als Derivate der 13-Thiaprostansäure bezeichnet werden.

In den vorstehenden Formeln bedeutet $R^1$ Wasserstoff oder eine solvolytisch oder hydrogenolytisch abspaltbare Schutzgruppe. Dabei handelt es sich vorzugsweise um z. B. gesättigte oder ungesättigte aliphatische, cycloaliphatische oder aromatische, substituierte oder unsubstituierte Carbon- oder Sulfonsäurereste oder auch einen anorganischen Säurerest. Bevorzugte Carbonsäurereste leiten sich von Fettsäuren ab, die 1 bis 18, vorzugsweise 1 bis 6 C-Atome besitzen, wie Ameisen-, Essig-, Butter- oder Isobuttersäure, aber z. B. auch Pivalin-, Trichloressig-, Benzoe-, p-Nitrobenzoe-, Palmitin-, Stearin- oder Ölsäure. Bevorzugte Sulfonsäurereste leiten sich ab von Alkylsulfonsäuren mit 1 bis 6 C-Atomen, z. B. Methan- oder Äthansulfonsäure, oder Arylsulfonsäuren mit 6 bis 10 C-Atomen, z. B. Benzol-, p-Toluol-, 1- und 2-Naphthalinsulfonsäure, auch von substituierten Sulfonsäuren wie 2-Hydroxyäthan- oder 4-Brombenzolsulfonsäure. Bevorzugte anorganische Säuren sind Schwefelsäure und Phosphorsäure.

Weiterhin können die Schutzgruppen auch Ätherreste sein, so daß die Hydroxygruppen in verätherter Form vorliegen, z. B. Aralkoxy mit vorzugsweise 7 bis 19 C-Atomen, wie Benzyloxy, p-Methylbenzyloxy, 1- und 2-Phenyläthoxy, Diphenylmethoxy, Triphenylmethoxy oder 1- oder 2-Naphthylmethoxy; Alkoxy mit vorzugsweise bis zu 6 C-Atomen, insbesondere tert.-Butoxy; Tetrahydropyranyloxy; oder Trialkylsilyloxy, vorzugsweise Trimethylsilyloxy oder tert.-Butyldimethylsilyloxy.

Da diese Schutzgruppen in den pharmakologisch wirksamen Verbindungen der Formel III nicht mehr erscheinen, ist ihre Natur an sich unkritisch.

$R^2$ bedeutet neben Wasserstoff auch Alkyl mit 1—3 C-Atomen, vorzugsweise unverzweigt, wie Methyl, Äthyl oder Propyl; aber auch verzweigt wie Isopropyl.

$R^3$ bedeutet einen Alkylrest mit 3—5 C-Atomen, vorzugsweise einen unverzweigten Alkylrest mit 3—5 C-Atomen wie Propyl, Butyl oder Pentyl; andere Alkylreste $R^3$ können sein: Isopropyl, Isobutyl, Isopentyl, sec.-Butyl, tert.-Butyl, Pent-2-yl, Pent-3-yl, 2-Methylbutyl oder Neopentyl.

$R^3$ bedeutet auch vorzugsweise Phenyl oder ein-, zwei- oder dreifach durch F, Cl, OH, $OCH_3$, $OC_2H_5$, $CF_3$ oder Alkyl mit 1—3 C-Atomen substituiertes Phenyl. Wenn $R^3$ ein substituierter Phenylrest ist, so ist er vorzugsweise einfach substituiert, wobei der Substituent in 2-Stellung, insbesondere aber in 3- oder 4-Stellung zu finden ist.

$R^3$ ist daher vorzugsweise auch 3-Fluorphenyl, 4-Fluorphenyl, 3-Chlorphenyl, 4-Chlorphenyl, 3-Hydroxyphenyl, 4-Hydroxyphenyl, 3-Methoxyphenyl, 4-Methoxyphenyl, 3-Äthoxyphenyl, 4-Äthoxyphenyl, 3-Trifluormethylphenyl, 4-Trifluormethylphenyl, 2-Tolyl, 3-Tolyl, 4-Tolyl, 4-Äthylphenyl, 4-Isopropylphenyl, aber auch beispielsweise 2,4-Dichlor-, 3,4-Dichlor-, 2,4-Dimethyl-, 3,4-Dimethyl-, 2,4-Dimethoxy-, 2,3-Dimethoxy-, 2,4,6-Trimethyl- oder 3,4,5-Trimethoxyphenyl.

$R^3$ bedeutet ferner, wenn A nicht $-CH_2O-$ ist, Methoxy, Äthoxy, Propyloxy oder Butyloxy, 2-Pyridyl, 3-Pyridyl, 4-Pyridyl, 2-Thienyl, 3-Thienyl, 1-Naphthyl oder 2-Naphthyl.

A bedeutet vorzugsweise $-CH_2-$, $-CH_2-CH_2-$ oder $-CH_2O-$. Daneben bedeutet A aber auch noch $-H(CH_3)-$, $-C(CH_3)_2-$, $-CH(CH_3)-CH_2-$, $-C(CH_3)_2CH_2-$ oder $-CH_2CH_2-CH_2-$.

M steht vorzugsweise für H, Na, K, 1/2 Ca oder $NH_4$. Daneben kann M auch ein Äquivalent eines anderen Metallatoms, vorzugsweise eines Alkali- oder Erdalkalimetallatoms oder ein durch 1—4 Alkylreste mit vorzugsweise 1—6 C-Atomen, durch Cycloalkylreste mit vorzugsweise 5—7 C-Atomen oder durch Aralkylreste mit vorzugsweise 7—11 C-Atomen substituiertes Ammoniumion bedeuten.

Wenn D eine –CHOH-Gruppe bedeutet, so kann die OH-Gruppe α- oder β-ständig sein.

Wenn E einen 1,2-Vinylenrest bedeutet, so ist dieser vorzugsweise cis-substituiert.

$R^4$ bedeutet insbesondere Wasserstoff, aber auch einen Alkylrest, vorzugsweise einen unverzweigten mit bis zu 4 C-Atomen, wie Methyl, Äthyl, Propyl oder n-Butyl, aber auch einen verzweigten, wie Isopropyl oder tert.-Butyl. Besonders vorteilhaft bedeutet $R^4$ auch den Rest –Q–NH–$COR^5$.

Q ist vorzugsweise 1,4-Phenylen, kann aber auch 1,4- Naphthylen bedeuten.

Bevorzugte Bedeutungen von $R^5$ sind Methyl, $NH_2$, 4-Acetylaminophenyl und insbesondere Phenyl.

$R^6$ ist ein Alkylrest mit 1–4 C-Atomen, vorzugsweise ein unverzweigter Alkylrest wie Methyl, Äthyl, Propyl oder Butyl, aber auch ein verzweigter Alkylrest mit 1–4 C-Atomen wie Isopropyl, sec.-Butyl, Isobutyl oder tert.-Butyl; vor allem ist $R^6$ aber eine Phenylgruppe. In der Regel sind alle drei Reste $R^6$ gleich, sie können aber auch ungleich sein. Sofern $R^6$ einen verzweigten Alkylrest bedeutet, sollen nicht mehr als 2 verzweigte Alkylreste, vorteilhafterweise nur 1 verzweigter Alkylrest mit dem P-Atom verknüpft sein. X bedeutet Cl, Br oder J, wobei Br bevorzugt ist.

Besonders bevorzugt sind diejenigen Verbindungen der Formel I, in denen mindestens eines der Symbole A, $R^1$, $R^2$ und $R^3$ eine der vorstehend als bevorzugt angegebenen Bedeutungen hat. Einige dieser bevorzugten Gruppen von Verbindungen können durch die nachstehenden Teilformeln Ia bis Ie gekennzeichnet werden, die sonst der Formel I entsprechen, und in denen die nicht näher bezeichneten Symbole die bei der Formel I angegebene Bedeutung haben, worin jedoch

in Ia A = $-CH_2-$, $-CH(CH_3)-$ oder $-C(CH_3)_2-$ und $R^3$ = Butyl,

in Ib A = $-CH_2-$, $-CH(CH_3)-$ oder $-C(CH_3)-$, $R^2$ = Methyl und $R^3$ = Butyl,

in Ic A = $-CH_2-CH_2-$ oder $-CH_2O-$ und $R^3$ = Phenyl, 3-Chlorphenyl, 3-Trifluormethylphenyl oder 3-Methoxyphenyl,

in Id A = $-CH_2-CH_2-$ oder $-CH_2O-$, $R^2$ = H oder Methyl und $R^3$ = Phenyl, 3-Chlorphenyl, 3-Trifluormethylphenyl oder 3-Methoxyphenyl,

in Ie A = $-CH_2CH_2-$ oder $-CH_2O-$, $R^1$ = H, $R^2$ = H oder Methyl und $R^3$ = Phenyl, 3-Chlorphenyl, 3-Trifluormethylphenyl oder 3-Methoxyphenyl.

Das 2-Brom-3-hydroxy-bicyclo(3.2.0)heptan-6-on, das aus Cyclopentadien gewonnen wird, ist beispielsweise beschrieben in J.C.S. Chem. Comm. 1974, Seite 948.

In den Verbindungen der Formel II haben die Reste A, $R^2$ und $R^3$ die vorstehend angegebenen, insbesondere die als bevorzugt genannten Bedeutungen. Es handelt sich bei den Verbindungen der Formel II um 2-Hydroxythiole oder deren Alkalimetall-, Erdalkalimetall- oder Ammoniumsalze. Die meisten Thiole der Formel II sind bekannt, beispielsweise aus der DE-OS 2 256 537, der DE-OS 2 422 924 und der DE-OS 2 644 972. Neue Verbindungen der Formel II können aus bekannten Verbindungen in Analogie zu bekannten Verfahren hergestellt werden, beispielsweise aus den entsprechenden Oxiranen durch Umsetzen mit $H_2S$ und gegebenenfalls anschließende Überführung in ihre Alkalimetall-, Erdalkalimetall- oder Ammoniumsalze. Ebenso können die Oxirane direkt mit Alkalimetall-, Erdalkalimetall- oder Ammoniumhydrogensulfiden umgesetzt werden, wobei dann direkt die Verbindungen der Formel II mit M ungleich H erhalten werden.

Die Umsetzung des 2-Brom-3-hydroxy-bicyclo(3.2.0)heptan-6-ons mit einem Thiol oder Thiolat der Formel II erfolgt in der Regel in Gegenwart eines basischen Katalysators und in Anwesenheit oder Abwesenheit eines inerten Lösungsmittels bei Temperaturen zwischen etwa –20 und +100°, vorzugsweise zwischen 0 und 30°. Als Lösungsmittel eignen sich vorzugsweise Alkohole wie Methanol, Äthanol, Propanol, Isopropanol, Butanol, tert.-Butylalkohol, Amylalkohol, 2-Methoxyäthanol oder 2-Äthoxyäthanol; Äther wie Diäthyläther, Tetrahydrofuran (THF), Dioxan oder Äthylenglykoldimethyläther; Kohlenwasserstoffe wie Benzol oder Toluol; halogenierte Kohlenwasserstoffe wie Methylenchlorid oder Chloroform oder auch Wasser.

Geeignete basische Katalysatoren sind z. B. Alkalimetall- oder Erdalkalimetallhydroxide wie NaOH, KOH oder $Ca(OH)_2$; Alkalimetallalkoholate, wie $NaOCH_3$, $NaOC_2H_5$ oder KO–tert.-$C_4H_9$; basische Salze, vorzugsweise Carbonate oder Acetate wie $K_2CO_3$ oder $NaOCOCH_3$; Ammoniak; Amine, vorzugsweise sekundäre oder tertiäre Amine wie Triäthylamin, Diisopropylamin, Dicyclohexylamin, Dimethylanilin, Piperidin, 2,6-Dimethylpiperidin, 2,2,6,6-Tetramethylpiperidin, Pyrrolidin, Pyridin, Chinolin, Diazabicyclo-[2,2,2]-octan oder Diazabicyclo-[3,4,0]-nonen; aber auch primäre Amine wie tert.-Butylamin oder Cyclohexylamin; oder quartäre Ammoniumhydroxide wie Tetramethylammoniumhydroxid oder Benzyltrimethylammoniumhydroxid. Es ist besonders vorteilhaft, eines der genannten Amine, insbesondere ein sekundäres oder tertiäres Amin, gleichzeitig als Lösungsmittel zu verwenden und so in Abwesenheit eines inerten Lösungsmittels zu arbeiten.

Die Einführung einer solvolytisch oder hydrogenolytisch abspaltbaren Schutzgruppe erfolgt durch Umsetzen einer Verbindung der Formel I mit $R^1$ = H mit einem veresternden oder veräthernden Mittel. Veresternde Mittel sind beispielsweise die bereits erwähnten Carbon-, Sulfon- oder anorganischen Säuren; veräthernde Mittel sind beispielsweise die bereits erwähnten aromatischen Alkohole mit 7 bis 19 C-Atomen, aliphatischen Alkohole mit bis zu 6 C-Atomen, Tetrahydropyran und Trialkylsilane.

Die Einführung einer solchen Schutzgruppe erfolgt zweckmäßig in einem inerten, vorzugsweise wasserfreien Lösungsmittel, beispielsweise einem Äther wie Diäthyläther oder THF, einem Alkohol, vorzugsweise mit bis zu 4 C-Atomen oder auch in einem Kohlenwasserstoff, wie Petroläther, Hexan, Benzol oder Toluol, oder in Gemischen dieser Lösungsmittel, vorzugsweise in Gegenwart einer anorganischen oder organischen Säure, wie HCl, HBr, HJ, $H_2SO_4$, $H_3PO_4$, Trifluoressigsäure, einer Sulfonsäure wie Benzolsulfonsäure oder p-Toluolsulfonsäure, oder eines sauren Ionenaustauschers bei Temperaturen zwischen −10° und 40°, vorzugsweise bei Raumtemperatur. Die Reaktionszeiten liegen in der Regel zwischen 30 Minuten und 20 Stunden.

Die Verbindungen der Formel I sind wertvolle Zwischenprodukte zur Herstellung von 13-Thiaprostaglandin-Derivaten der Formel III.

Im Rahmen dieser Synthese werden die Verbindungen der Formel I mit ultraviolettem Licht vorzugsweise der Wellenlänge $\lambda$= 280—350 nm bestrahlt. Diese Ultraviolett-Bestrahlung erfolgt in der Regel in wäßriger oder organischer Lösung bei Temperaturen zwischen 0 und 50°, vorzugsweise zwischen 10 und 30°. Die Bestrahlung kann mit Hilfe der verschiedensten handelsüblichen Ultraviolett-Geräte erfolgen. In der Regel bestrahlt man mit einer Quecksilbermitteldrucklampe durch ein Pyrexfilter. Die Bestrahlungszeit beträgt in der Regel zwischen 2 und 100 Stunden, vorzugsweise zwischen 5 und 50 Stunden, insbesondere zwischen 10 und 20 Stunden. Als Lösungsmittel verwendet man zweckmäßig Wasser oder Alkohole mit 1—5 C-Atomen wie Methanol, Äthanol, Propanol, Isopropanol, Butanol, tert.-Butylalkohol oder Amylalkohol, ferner Gemische von Alkoholen mit Wasser, von Äthern, wie Diäthyläther oder THF, mit Wasser, von Alkoholen mit Estern wie Essigester, Ketonen wie Aceton, Amiden wie DMF oder Kohlenwasserstoffen wie Benzol, Toluol. Es empfiehlt sich, kleine Mengen von Butadien-Derivaten, wie 2,5-Dimethyl-2,4-hexadien, 1,4-Di-tert.-butyl-1,3-butadien, dem Reaktionsansatz zuzusetzen. Führt man die Ultraviolett-Bestrahlung in alkoholischer Lösung durch, so muß anschließend in Gegenwart eines sauren Katalysators hydrolysiert werden. Die Hydrolysezeiten liegen zwischen etwa einer Stunde und ca. 48 Stunden; man arbeitet bei Temperaturen zwischen etwa −5° und 80°, vorzugsweise bei Raumtemperatur. Als hydrolysierende Mittel verwendet man vorzugsweise Wasser oder Wasser im Gemisch mit organischen Lösungsmitteln. Als organische Lösungsmittel können Alkohole wie Methanol, Äthanol, Propanol, Isopropanol, Butanol, tert.-Butylalkohol, Amylalkohol, 2-Methoxyäthanol oder 2-Äthoxyäthanol; Äther wie Diäthyläther, THF, Dioxan oder 1,2-Dimethoxyäthan; Säuren wie Ameisensäure, Essigsäure, Propionsäure oder Buttersäure; Ester wie Äthylacetat oder Butylacetat; Ketone wie Aceton; Amide wie Dimethylformamid (DMF) oder Hexamethylphosphorsäuretriamid (HMPT); Nitrile wie Acetonitril; Sulfoxide wie Dimethylsulfoxid (DMSO); Sulfone wie Tetrahydrothiophen-S,S-dioxid verwendet werden.

Als saure Katalysatoren eignen sich anorganische Säuren, beispielsweise Salz-, Schwefel-, Phosphor- oder Bromwasserstoffsäure; organische Säuren, wie Chloressigsäure, Trichloressigsäure oder Trifluoressigsäure, Methan-, Äthan-, Benzol- oder p-Toluolsulfonsäure oder saure anorganische Salze wie $MgSO_4$, $Al_2(SO_4)_3$, $CaCl_2$.

Die erhaltenen Lactole der Formel IV können anschließend mit den Verbindungen der Formel V zu den 13-Thia-Prostaglandin-F-Analogen der Formel III umgesetzt werden. Die Verbindungen der Formel V sind bekannt, beispielsweise aus der DE-OS 2 431 930, der DE-OS 2 644 972 und aus Tetrahedron Letters 1970, Heft 4, Seiten 311—313.

Die Umsetzung eines Lactols der Formel IV mit einer Verbindung der Formel V ist bekannt, beispielsweise aus der DE-OS 2 550 004 und der DE-OS 2 644 972. Sie wird zweckmäßigerweise in einem inerten organischen Lösungsmittel durchgeführt. Kohlenwasserstoffe wie Cyclohexan, Toluol, Xylol und insbesondere Benzol sind bevorzugt, ebenso Acetonitril; aber z. B. auch Äther wie Diäthyläther, Diisopropyläther, 1,2-Dimethoxyäthan oder THF sind geeignete Reaktionsmedien. Die Reaktionstemperaturen liegen etwa zwischen −40° und 150°; vorzugsweise arbeitet man bei Raumtemperatur.

Die Umsetzung einer Verbindung der Formel IV mit eine Verbindung der Formel V erfolgt in Analogie zur Herstellung bekannter Verbindungen wie $PGF_{2\alpha}$ oder 2,3-trans-Methano-11,15-bis-tetrahydropyranylprostaglandin $F_{2\alpha}$ nach literaturbekannten, z. B. in der DE-OS 2 431 930 beschriebenen Standardmethoden, beispielsweise durch Wittig-Reaktion (wobei man in Gegenwart einer starken Base, beispielsweise eines Alkalimetallhydrids wie NaH oder einer Lithiumalkylverbindung wie Butyllithium, arbeitet), vorzugsweise in Dimethylsulfoxid (DMSO) als Lösungsmittel bei Temperaturen zwischen etwa 15° und etwa 80°. Besonders zweckmäßig ist es, unter einer Inertgasatmosphäre, beispielsweise unter Stickstoff, zu arbeiten. In den so erhaltenen Verbindungen der Formel I bedeutet E eine cis-C=C-Doppelbindung.

Eine Verbindung der Formel III mit E = —CH=CH— kann in die entsprechenden Verbindungen mit E = $-CH_2-CH_2-$ überführt werden, z. B. durch katalytische Hydrierung. Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat), als Oxide (z. B. Platinoxid) oder in feinteiliger Form vorliegen können. Nickel- und Kobaltkatalysatoren werden zweckmäßig als Raney-Metalle eingesetzt. Man kann zweckmäßig bei Drücken zwischen etwa 1 und 200 at und bei Temperaturen zwischen etwa −80 und +150°, vorzugsweise bei Raumtemperatur, hydrieren. Die Hydrierung erfolgt in Gegenwart eines inerten Lösungsmittel, z. B. eines Alkohols wie Methanol, Äthanol oder Isopropanol, einer Carbonsäure wie Essigsäure, eines Esters wie Äthylacetat, eines Äthers wie Tretrahydrofuran oder Dioxan, eines Ketons wie Aceton. Man kann auch Lösungsmittelgemische verwenden,

z. B. auch Wasser enthaltende Gemische.

Falls erwünscht, kann eine erhaltene Verbindung der Formel III mit D = —CHOH— in eine Verbindung der Formel III mit D = —CO— überführt werden. Als oxidierende Mittel, mit welchen es gelingt, eine Gruppe D = —CHOH— in eine andere Gruppe D = —CO— umzuwandeln, kommen vor solche in Frage, die im basischen, neutralen oder auch schwach sauren Milieu oxidierend wirken. Man verwendet beispielsweise Ketone, vorzugsweise aliphatische oder cycloaliphatische wie Aceton oder Cyclohexanon, aber auch Chinone wie 1,4-Benzochinon in Gegenwart eines Aluminiumalkoxids, vorzugsweise Aluminium-tri-tert.-butoxid oder Aluminium-tris-isopropoxid (Oppenauer-Oxidation), Sulfoxide, vorzugsweise Dimethylsulfoxid, gegebenenfalls in Gegenwart eines weiteren Hilfsstoffes, beispielsweise eines Carbodiimids, wie Dicyclohexylcarbodiimid, eines Salzes einer organischen Base wie Pyridiniumphosphat, Pyridiniumsulfat, Pyridiniumchlorid oder Pyridiniumtrifluoracetat (Pfitzner-Moffatt-Oxidation), N-Halogenamide bzw. N-Halogenimide, vorzugsweise N-Bromacetamid, N-Bromsuccinimid oder N-Chlor-succinimid, gegebenenfalls in Gegenwart weiterer Hilfsstoffe wie Pyridin, Aceton, organischen Sulfiden, wie Dimethylsulfid oder Thioanisol, Chlor in Gegenwart eines organischen Sulfids, beispielsweise Thioanisol, organische Hypochlorite, vorzugsweise tert.-Butylhypochlorit, gegebenenfalls in Gegenwart eines vorzugsweise tertiären Amins wie Pyridin. Ferner kommen als oxidierende Mittel in Frage Chromtrioxid in Gegenwart von Pyridin (Collins-Reagens) oder in Gegenwart von verdünnter Schwefelsäure (Jones-Reagens), Edelmetallsalze oder -oxide wie $PdCl_2$, $RuO_4$, $OsO_4$. Oxidationen der Gruppe D = —CHOH— zu —CO— sollen unter relativ milden Reaktionsbedingungen ausgeführt werden. Die Reaktionstemperaturen liegen etwa zwischen —30° und +60°, vorzugsweise zwischen —15° und Raumtemperatur; die Reaktionszeiten betragen im allgemeinen 30 Minuten bis 4 Stunden.

Gegebenenfalls vorhandene Hydroxy-Schutzgruppen in $C_{11}$- und/oder $C_{15}$-Stellung können solvolytisch oder hydrogenolytisch abgespalten werden. Die Umsetzung mit solvolysierenden Mitteln wird z. B. bei Temperaturen zwischen —20° und 40° ausgeführt. In der Regel arbeitet man in Gegenwart eines sauren, vorzugsweise eines basischen Katalysators unter Verwendung eines inerten Lösungsmittels.

Solvolysierende Mittel sind vorzugsweise hydrolysierende Mittel wie reines Wasser oder Wasser im Gemisch mit organischen Lösungsmitteln, meist in Gegenwart eines sauren oder basischen Katalysators. Als organische Lösungsmittel kommen z. B. in Frage Alkohole wie Methanol, Äthanol, Propanol, Isopropanol, Butanol, tert.-Butylalkohol, Amylalkohol, 2-Methoxyäthanol oder 2-Äthoxyäthanol; Äther wie Diäthyläther, THF, Dioxan oder Äthylenglykoldimethyläther; Säuren wie Ameisensäure, Essigsäure, Propionsäure oder Buttersäure; Ester wie Äthylacetat oder Butylacetat; Ketone wie Aceton; Amide wie Dimethylformamid (DMF) oder Hexamethylphosphorsäuretriamid (HMPT); Nitrile wie Acetonitril; Sulfoxide wie DMSO; Sulfone wie Tetrahydrothiophen-S,S-dioxid; sowie Gemische dieser Lösungsmittel.

Als saure Katalysatoren eignen sich bei einer Solvolyse anorganische Säuren, beispielsweise Salz-, Schwefel-, Phosphor- oder Bromwasserstoffsäure; organische Säuren, wie Chloressigsäure, Trichloressigsäure oder Trifluoressigsäure, Methan-, Äthan-, Benzol- oder p-Toluolsulfonsäure. Als basische Katalysatoren verwendet man bei einer Solvolyse zweckmäßig Alkalimetall- oder Erdalkalimetallhydroxide wie Natrium-, Kalium- oder Calciumhydroxid, oder basische Salze, wie Natrium- oder Kaliumcarbonat. Auch organische Basen, beispielsweise Äthyl-, Diäthyl-, Triäthyl-, Isopropyl-, n-Butyl- oder Tri-n-butylamin, Äthanolamin, Triäthanolamin, Cyclohexylamin, Dimethylanilin, Pyrrolidin, Piperidin, Morpholin, Pyridin, $\alpha$-Picolin oder Chinolin; oder quartäre Ammoniumhydroxide, wie z. B. Tetramethylammoniumhydroxid oder Benzyltrimethylammoniumhydroxid können als basische Katalysatoren verwendet werden. Ein Überschuß des Katalysators kann auch an Stelle eines Lösungsmittels verwendet werden.

Die Solvolysezeiten liegen zwischen etwa einer Stunde und ca. 48 Stunden; man arbeitet bei Temperaturen zwischen etwa —5° und ca. 80°, vorzugsweise bei etwa Raumtemperatur.

Hydrogenolysierendes Mittel ist insbesondere katalytisch angeregter Wasserstoff.

Hydrogenolytische Abspaltungen von Schutzgruppen, beispielsweise von Benzylgruppen mit katalytisch angeregtem Wasserstoff, erfolgen bei an sich bekannten Reaktionsbedingungen, vorzugsweise in Gegenwart eines Metallkatalysators, wie Raney-Nickel oder Raney-Cobalt, vor allem in Gegenwart eines Edelmetallkatalysators, wie Pt oder Pd, gegebenenfalls unter Verwendung eines Trägers wie Kohlenstoff oder $CaSO_4$; man kann auch Oxidkatalysatoren, beispielsweise $PtO_2$, verwenden. Geeignete Lösungsmittel zur Durchführung der hydrogenolytischen Abspaltung sind z. B. Alkohole wie Methanol oder Äthanol, Carbonsäuren wie Ameisen- oder Essigsäure, Ester wie Äthylacetat oder Äthylbutyrat, Ketone wie Aceton, Äther wie THF bzw. Gemische dieser Lösungsmittel. Die Hydrogenolysen werden vorzugsweise zwischen etwa Raumtemperatur und ca. 40° durchgeführt.

Ein Ester der Formel III ($R^4$ = Alkyl mit 1 bis 4 C-Atomen) kann aus einer Säure der Formel I ($R^4$ = H) durch Umsetzen mit einem veresternden Mittel hergestellt werden. Veresternde Mittel sind beispielsweise Alkohole mit bis zu 4 C-Atomen, vorzugsweise in Gegenwart einer anorganischen oder organischen Säure, wie HCl, HBr, HJ, $H_2SO_4$, $H_3PO_4$, Trifluoressigsäure, einer Sulfonsäure wie Benzolsulfonsäure oder p-Toluolsulfonsäure, oder eines sauren Ionenaustauschers; Diazoalkane mit bis zu 4 C-Atomen, vorzugsweise Diazomethan; Olefine (z. B. Isobutylen), vorzugsweise in Gegenwart von sauren Katalysatoren (z. B. $ZnCl_2$, $BF_3$, $H_2SO_4$, Arylsulfonsäure, Pyrophosphorsäure, Borsäure, Oxalsäure); Alkylhalogenide mit bis zu 4 C-Atomen, vorzugsweise Bromide, wie Äthyl-, Propyl-, Isopropyl- oder

Butylbromid, aber auch die entsprechenden -chloride oder -jodide; Carbonsäure- oder Sulfonsäurealkylester, wobei der Säurerest beliebig sein kann und der Alkylrest bis zu 4 C-Atome enthält, vorzugsweise Methyl-, Äthyl-, Propyl-, Isopropyl- oder Butylacetat, -formiat, -methylsulfonat, -äthylsulfonat oder -p-toluolsulfonat; und insbesondere auch Dialkylschwefelsäureester mit bis zu 4 C-Atomen, wie Dimethylsulfat oder Diäthylsulfat.

Die Veresterung erfolgt zweckmäßig in einem inerten, vorzugsweise wasserfreien Lösungsmittel, beispielsweise einem Äther wie Diäthyläther oder THF, einem Alkohol, vorzugsweise einem der genannten Alkohole mit bis zu 4 C-Atomen oder auch in einem Kohlenwasserstoff, wie Petroläther, Hexan, Benzol oder Toluol, oder in Gemischen dieser Lösungsmittel bei Temperaturen zwischen −10° und 40°, vorzugsweise bei Raumtemperatur. Die Reaktionszeiten liegen in der Regel zwischen 30 Minuten und 20 Stunden.

Ein Ester der Formel III ($R^4$ = —Q—NH—$COR^5$) kann aus einer Säure der Formel III ($R^4$ = H) durch Umsetzen mit einer Verbindung der Formel VI HO—Q—NH—$COR^5$ (worin Q und $R^5$ die oben angegebenen Bedeutungen haben) hergestellt werden. Die Verbindungen der Formel VI sind bekannt, beispielsweise aus der DE-OS 2 453 271 und der DE-OS 2 644 972. Es handelt sich vorzugsweise um Derivate des p-Aminophenols.

Die Umsetzung einer Säure der Formel III ($R^4$ = H) mit einer Verbindung der Formel VI kann nach an sich bekannten Methoden durchgeführt werden; vorzugsweise arbeitet man in Gegenwart eines wasserbindenden Mittels, z. B. eines Carbodiimids, wie Dicyclohexylcarbodiimid, und in einem inerten organischen Lösungsmittel, vorzugsweise einem Äther wie Diäthyläther, 1,2-Dimethoxyäthan, Tetrahydrofuran (THF) oder Dioxan; oder einem Halogenkohlenwasserstoff wie Methylenchlorid oder 1,2-Dichloräthan; oder in Gemischen dieser Lösungsmittel mit DMF. Reaktionsbedingungen, welche für diese Umsetzung geeignet sind, sind bekannt und z. B. in Tetrahedron 21, 3531 (1965), oder in Tetrahedron Letters 1978, 4475, beschrieben; die Reaktionstemperaturen liegen beispielsweise etwa zwischen 20° und 100°.

Da die Ester der Formel III mit $R^4$ = —Q—NH—$COR^5$ gut kristallisieren, können sie auch vorteilhaft zur Reinigung von 13-Thiaprostaglandinen mit freier Carboxylgruppe verwendet werden, welche im allgemeinen als schwer zu reinigende Öle erhalten werden. Nach Überführung dieser Öle in die gut kristallisierenden Ester der Formel III mit $R^4$ = —Q—NH—$COR^5$ können diese einfach und in an sich bekannter Weise aus üblichen Lösungsmitteln umkristallisiert werden.

Ester der Formel III ($R^4$ = Alkyl mit 1—4 C-Atomen oder —Q—NH—$COR^5$) können durch Hydrolyse in eine Säure der Formel III ($R^4$ = H) umgewandelt werden. Bevorzugt ist die basische Hydrolyse zu den Säuren der Formel III.

Man arbeitet vorzugsweise in wässerigen Medien, beispielsweise in Gemischen von Wasser mit Alkoholen, vorzugsweise niederen Alkanolen, wie Methanol oder Äthanol, oder mit Äthern, wie Äthylenglykolmonomethyläther, Äthylenglykoldimethyläther, THF oder Dioxan bei Temperaturen zwischen 0° und 40°, vorzugsweise bei Raumtemperatur. Die Reaktionszeiten betragen ungefähr eine Stunde bis 12 Stunden.

Die Verbindungen der Formel III werden meist als Gemische verschiedener stereoisomerer Formen erhalten, d. h. in der Regel als Gemische von Racematen. Racemate können aus den Racematgemischen isoliert und rein erhalten werden, beispielsweise durch Umkristallisieren der Verbindungen selbst oder von gut kristallisierenden Derivaten, insbesondere aber mit Hilfe chromatographischer Methoden, wobei sowohl adsorptionschromatographische oder verteilungschromatographische Methoden als auch Mischformen in Frage kommen.

Die Racemate können nach bekannten Methoden, wie sie in der Literatur angegeben sind, in ihre optischen Antipoden getrennt werden. Die Methode der chemischen Trennung wird bevorzugt.

Weiterhin ist es natürlich möglich, optisch aktive Verbindungen nach den beschriebenen Methoden zu erhalten, indem man Ausgangsstoffe verwendet, die bereits optisch aktiv sind.

Die Verbindungen der Formel III können mit mindestens einem festen, flüssigen und/oder halbflüssigen, in der Pharmazie üblichen Träger- oder Hilfsstoff vermischt werden. Die Gemische der Verbindungen der Formel III mit den in der Pharmazie üblichen Träger- oder Hilfsstoffen können als Arzneimittel in der Human- oder Veterinärmedizin verwendet werden. Als Trägerstoffe kommen solche organischen oder anorganischen Stoffe in Frage, die für die parenterale, enterale (z. B. orale) oder topikale Applikation geeignet sind und mit den Verbindungen der Formel III nicht reagieren, beispielsweise Wasser, pflanzliche Öle, Benzylalkohole, Polyäthylenglykole, Glycerintriacetat, Gelatine, Lactose, Stärke, Magnesiumstearat, Talk, Vaseline, Cholesterin. Für die orale Applikation eignen sich Tabletten, Dragees, Kapseln, Sirupe, Säfte oder Tropfen; zur rektalen Anwendung Suppositorien; zur parenteralen Anwendung Lösungen, vorzugsweise ölige oder wässerige Lösungen, ferner Suspensionen, Emulsionen oder Implantate; für die topikale Anwendung Salben, Cremes oder Puder.

Die Verbindungen der Formel III können auch lyophilisiert und die erhaltenen Lyophilisate z. B. zur Herstellung von Injektionspräparaten verwendet werden. Die angegebenen Zubereitungen können gegebenenfalls sterilisiert oder mit Hilfsstoffen wie Gleit-, Konservierungs-, Stabilisierungs- oder Netzmitteln, Emulgatoren, Salzen zur Beeinflussung des osmotischen Druckes, Puffersubstanzen, Farb-, Geschmacks- und/oder Aromastoffen versetzt werden. Sie können, falls erwünscht, auch einen oder mehrere weitere Wirkstoffe enthalten, z. B. ein oder mehrere Vitamine.

Die Verbindungen der Formel III werden vorzugsweise in einer Dosierung von 0,01 bis 200 mg pro Dosierungseinheit verabreicht; die Dosierung ist abhängig von der behandelten Spezies, der Applikationsform und dem Behandlungszweck, sie kann daher die oben angegebenen Werte auch unter- bzw. überschreiten.

Will man beispielsweise die östrussynchronisierende Wirkung der Verbindungen der Formel III ausnützen, so ist es besonders vorteilhaft, z. B. Rindern (Kühe bzw. Färsen) etwa 0,1 mg bis etwa 30 mg, vorzugsweise etwa 0,5 mg bis etwa 20 mg, insbesondere etwa 1,5 mg bis etwa 15 mg des Wirkstoffes intramuskulär zu injizieren. Es ist günstig, die wirksame Dosis durch einmalige Injektion zwischen etwa dem 7. Tag und etwa dem 12. Tag des Zyklus zu verabreichen, man kann aber auch mehrmals und gegebenenfalls über mehrere Tage verteilt Teildosen ode jeweils die wirksame Dosis an zwei verschiedenen Tagen, z. B. am 1. und am 3. Tag, injizieren. Auch bei anderen Nutztieren, beispielsweise bei Hunden, Pferden, Schafen und Schweinen, kann der Östrus durch Verabreichung einer Verbindung der Formel III synchronisiert werden. Die wirksame Dosis variiert dabei in Abhängigkeit vom mittleren Körpergewicht der behandelten Spezies und kann unschwer vom Fachmann mit Hilfe der oben für Rinder angegebenen Richtwerte ermittelt werden.

IR-Spektren (IR) wurden durch Angabe der Hauptbanden charakterisiert (als Film).

Die NMR-Spektren (NMR) wurden in $CDCl_3$ gegen Tetramethylsilan gemessen und durch Angabe der Signale in ppm charakterisiert.

## Beispiel 1

Man löst 102 g 3α-Hydroxy-2β-brom-1βH,5βH-bicyclo(3.2.0)heptan-6-on und 209 g 3-(3-Chlorphenoxy)-2-hydroxy-propylthiol in 500 ml Methanol und versetzt bei Raumtemperatur tropfenweise mit einer Lösung von 60 g Kaliumtertiärbutylat in 200 ml Methanol. Anschließend rührt man noch 5 Stunden weiter, säuert mit 5 ml Essigsäure an und destilliert das Lösungsmittel ab. Man teilt den Rückstand zwischen Wasser und Methylenchlorid, trennt die organische Phase ab, trocknet sie über $MgSO_4$, destilliert das Lösungsmittel ab und erhält nach einer Kurzfiltration über Kieselgel als farbloses Öl 3α-Hydroxy-2β-(3-(3-chlorphenoxy)-2-hydroxypropylthiol)-1βH,5βH-bicyclo(3.2.0)heptan-6-on, das aus Toluol bei −30°C auskristallisiert. Ausbeute 84 g.

IR: 1600, 1780, 2950 und 3400 $cm^{-1}$;
NMR: 3,6–3,8; 4,4; 6,8–7,4.

Analog Beispiel 1 sind aus 3-Hydroxy-2β-brom-1,5-bicyclo(3.2.0)heptan-6-on durch Umsetzen mit einem entsprechenden Thiol der Formel II die in den folgenden Beispielen 2 bis 30 genannten Verbindungen der Formel I erhältlich:

| Beispiel | Verbindung der Formel I |
|---|---|
| 2 | 3-Hydroxy-2β-(3-phenoxy-2-hydroxypropylthio)-1,5-bicyclo(3.2.0)heptan-6-on, |
| 3 | 3-Hydroxy-2β-(3-(4-fluorphenoxy)-2-hydroxypropylthio)-1,5-bicyclo(3.2.0)heptan-6-on, |
| 4 | 3-Hydroxy-2β-(3-(4-chlorphenoxy)-2-hydroxypropylthio)-1,5-bicyclo(3.2.0)heptan-6-on, |
| 5 | 3-Hydroxy-2β-(3-(4-bromphenoxy)-2-hydroxypropylthio)-1,5-bicyclo(3.2.0)heptan-6-on, |
| 6 | 3-Hydroxy-2β-(3-(4-hydroxyphenoxy)-2-hydroxypropylthio)-1,5-bicyclo(3.2.0)heptan-6-on, |
| 7 | 3-Hydroxy-2β-(3-(4-methoxyphenoxy)-2-hydroxypropylthio)-1,5-bicyclo(3.2.0)heptan-6-on, |
| 8 | 3-Hydroxy-2β-(3-(4-tolyloxy)-2-hydroxypropylthio)-1,5-bicyclo(3.2.0)heptan-6-on, |
| 9 | 3-Hydroxy-2β-(3-(4-trifluormethylphenoxy)-2-hydroxypropylthio)-1,5-bicyclo(3.2.0)-heptan-6-on, |
| 10 | 3-Hydroxy-2β-(3-(3-methoxyphenoxy)-2-hydroxypropylthio)-1,5-bicyclo(3.2.0)heptan-6-on, |
| 11 | 3-Hydroxy-2β-(3-(3-trifluormethylphenoxy)-2-hydroxypropylthio)-1,5-bicyclo(3.2.0)-heptan-6-on, |

Fortsetzung

| Beispiel | Verbindung der Formel I |
|---|---|
| 12 | 3-Hydroxy-2β-(3-(2,4-dichlorphenoxy)2-hydroxypropylthio)-1,5-bicyclo(3.2.0)heptan-6-on, |
| 13 | 3-Hydroxy-2β-(3-(2,4-dimethoxyphenoxy)-2-hydroxypropylthio)-1,5-bicyclo(3.2.0)-heptan-6-on, |
| 14 | 3-Hydroxy-2β-(3-(2,4,6-trimethylphenoxy)-2-hydroxypropylthio)-1,5-bicyclo(3.2.0)-heptan-6-on, |
| 15 | 3-Hydroxy-2β-(3-(3,4,5-trimethoxyphenoxy)-2-hydroxypropylthio)-1,5-bicyclo(3.2.0)-heptan-6-on, |
| 16 | 3-Hydroxy-2β-(3-phenoxy-2-hydroxy-2-methylpropylthio)-1,5-bicyclo(3.2.0)heptan-6-on, |
| 17 | 3-Hydroxy-2β-(3-(4-fluorphenoxy)-2-hydroxy-2-methylpropylthio)-1,5-bicyclo(3.2.0)-heptan-6-on, |
| 18 | 3-Hydroxy-2β-(3-(4-chlorphenoxy)-2-hydroxy-2-methylpropylthio)-1,5-bicyclo(3.2.0)-heptan-6-on, |
| 19 | 3-Hydroxy-2β-(3-(4-bromphenoxy)-2-hydroxy-2-methylpropylthio)-1,5-bicyclo(3.2.0)-heptan-6-on, |
| 20 | 3-Hydroxy-2β-(3-(4-hydroxyphenoxy)-2-hydroxy-2-methylpropylthio)-1,5-bicyclo(3.2.0)-heptan-6-on, |
| 21 | 3-Hydroxy-2β-(3-(4-methoxyphenoxy)-2-hydroxy-2-methylpropylthio)-1,5-bicyclo-(3.2.0)heptan-6-on, |
| 22 | 3-Hydroxy-2β-(3-(4-tolyloxy)-2-hydroxy-2-methylpropylthio)-1,5-bicyclo(3.2.0)heptan-6-on, |
| 23 | 3-Hydroxy-2β-(3-(4-trifluormethylphenoxy)-2-hydroxy-2-methylpropylthio)-1,5-bicyclo-(3.2.0)heptan-6-on, |
| 24 | 3-Hydroxy-2β-(3-(3-chlorphenoxy)-2-hydroxy-2-methylpropylthio)-1,5-bicyclo(3.2.0)-heptan-6-on, |
| 25 | 3-Hydroxy-2β-(3-(3-methoxyphenoxy)-2-hydroxy-2-methylpropylthio)-1,5-bicyclo-(3.2.0)heptan-6-on, |
| 26 | 3-Hydroxy-2β-(3-(3-trifluormethylphenoxy)-2-hydroxy-2-methylpropylthio)-1,5-bicyclo-(3.2.0)heptan-6-on, |
| 27 | 3-Hydroxy-2β-(3-(2,4-dichlorphenoxy)-2-hydroxy-2-methylpropylthio)-1,5-bicyclo-(3.2.0)heptan-6-on, |
| 28 | 3-Hydroxy-2β-(3-(2,4-dimethoxyphenoxy)-2-hydroxy-2-methylpropylthio)-1,5-bicyclo-(3.2.0)heptan-6-on, |
| 29 | 3-Hydroxy-2β-(3-(2,4,6-trimethylphenoxy)-2-hydroxy-2-methylpropylthio)-1,5-bicyclo-(3.2.0)heptan-6-on, |
| 30 | 3-Hydroxy-2β-(3-(3,4,5-trimethoxyphenoxy)-2-hydroxy-2-methylpropylthio)-1,5-bicyclo-(3.2.0)heptan-6-on. |

## Beispiel 31

Man legt 100 g 3α-Hydroxy-2β-brom-1βH,5βH-bicyclo(3.2.0)heptan-6-on und 100 g 2-Hydroxy-2-methyl-heptylthiol in 700 ml Methanol vor und versetzt tropfenweise mit einer Lösung von 135 g Kaliumtertiärbutylat in 300 ml Methanol. Man rührt noch 1 Stunde nach, säuert mit 5 ml Essigsäure an, destilliert das Lösungsmittel ab und nimmt den Rückstand in Methylenchlorid auf. Man wäscht die organische Phase zweimal mit Wasser, trocknet sie über $MgSO_4$ und destilliert das Lösungsmittel ab. Man behandelt den öligen Rückstand zweimal mit Petroläther, um überschüssiges Thiol zu entfernen, filtriert über eine kurze Kieselgel-Säule und erhält 3α-Hydroxy-2β-(2-hydroxy-2-methyl-heptylthio)-1βH,5βH-bicyclo(3.2.0)heptan-6-on. Ausbeute 79 g.

IR: 1780, 2950 und 3400 $cm^{-1}$;
NMR: 1,28; 2,8; 3,2; 3,6–3,8; 4,4.

Analog Beispiel 31 sind aus 3-Hydroxy-2β-brom-1,5-bicyclo(3.2.0)heptan-6-on durch Umsetzen mit einem entsprechenden Thiol der Formel II die in den folgenden Beispielen 32 bis 43 genannten Verbindungen der Formel I erhältlich:

| Beispiel | Verbindung der Formel I |
|---|---|
| 32 | 3-Hydroxy-2β-(2-hydroxy-heptylthio)-1,5-bicyclo(3.2.0)heptan-6-on, |
| 33 | 3-Hydroxy-2β-(2-hydroxy-3-methyl-heptylthio)-1,5-bicyclo(3.2.0)heptan-6-on, |
| 34 | 3-Hydroxy-2β-(2-hydroxy-2,3-dimethyl-heptylthio)-1,5-bicyclo(3.2.0)heptan-6-on, |
| 35 | 3-Hydroxy-2β-(2-hydroxy-3,3-dimethyl-heptylthio)-1,5-bicyclo(3.2.0)heptan-6-on, |
| 36 | 3-Hydroxy-2β-(2-hydroxy-3-äthyl-heptylthio)-1,5-bicyclo(3.2.0)heptan-6-on, |
| 37 | 3-Hydroxy-2β-(2-hydroxy-2-methyl-3-äthyl-heptylthio)-1,5-bicyclo(3.2.0)heptan-6-on, |
| 38 | 3-Hydroxy-2β-(2-hydroxy-4-äthyl-heptylthio)-1,5-bicyclo(3.2.0)heptan-6-on, |
| 39 | 3-Hydroxy-2β-(2-hydroxy-2-methyl-4-äthyl-heptylthio)-1,5-bicyclo(3.2.0)heptan-6-on, |
| 40 | 3-Hydroxy-2β-(2-hydroxy-2-methyl-5-methoxy-pentylthio)-1,5-bicyclo(3.2.0)heptan-6-on, |
| 41 | 3-Hydroxy-2β-(2-hydroxy-2-methyl-4-äthoxy-butylthio)-1,5-bicyclo(3.2.0)heptan-6-on, |
| 42 | 3-Hydroxy-2β-(2-hydroxy-2-methyl-3-propyloxy-propylthio)-1,5-bicyclo(3.2.0)heptan-6-on, |
| 43 | 3-Hydroxy-2β-(2-hydroxy-2-methyl-3-butyloxy-propylthio)-1,5-bicyclo(3.2.0)heptan-6-on. |

## Beispiel 44

Analog Beispiel 1 setzt man 3-Hydroxy-2β-brom-1,5-bicyclo(3.2.0)heptan-6-on mit 2-Phenyl-2-hydroxy-propylthiol in Gegenwart von Kaliumtertiärbutylat um und erhält 3-Hydroxy-2β-(2-phenyl-2-hydroxy-propylthio)-1,5-bicyclo(3.2.0)heptan-6-on.

Analog Beispiel 44 sind durch Umsetzen des 3-Hydroxy-2β-brom-1,5-bicyclo(3.2.0)heptan-6-on mit einem entsprechenden Thiol der Formel II die in den folgenden Beispielen 45 bis 73 genannten Verbindungen der Formel I erhältlich:

| Beispiel | Verbindung der Formel I |
|---|---|
| 45 | 3-Hydroxy-2β-(2-(4-fluorphenyl)-2-hydroxy-propylthio)-1,5-bicyclo(3.2.0)heptan-6-on, |
| 46 | 3-Hydroxy-2β-(2-(4-chlorphenyl)-2-hydroxy-propylthio)-1,5-bicyclo(3.2.0)heptan-6-on, |
| 47 | 3-Hydroxy-2β-(2-(4-bromphenyl)-2-hydroxy-propylthio)-1,5-bicyclo(3.2.0)heptan-6-on, |
| 48 | 3-Hydroxy-2β-(2-(4-hydroxyphenyl)-2-hydroxy-propylthio)-1,5-bicyclo(3.2.0)heptan-6-on, |
| 49 | 3-Hydroxy-2β-(2-(4-methoxyphenyl)-2-hydroxy-propylthio)-1,5-bicyclo(3.2.0)heptan-6-on, |
| 50 | 3-Hydroxy-2β-(2-(4-tolyl)-2-hydroxy-propylthio)-1,5-bicyclo(3.2.0)heptan-6-on, |
| 51 | 3-Hydroxy-2β-(2-(4-trifluormethylphenyl)-2-hydroxy-propylthio)-1,5-bicyclo(3.2.0)-heptan-6-on, |
| 52 | 3-Hydroxy-2β-(2-(3-methoxyphenyl)-2-hydroxy-propylthio)-1,5-bicyclo(3.2.0)heptan-6-on, |
| 53 | 3-Hydroxy-2β-(2-(3-chlorphenyl)-2-hydroxy-propylthio)-1,5-bicyclo(3.2.0)heptan-6-on, |
| 54 | 3-Hydroxy-2β-(2-(3-trifluormethylphenyl)-2-hydroxy-propylthio)-1,5-bicyclo(3.2.0)-heptan-6-on, |
| 55 | 3-Hydroxy-2β-(2-(3,4,5-trimethoxyphenyl)-2-hydroxy-propylthio)-1,5-bicyclo(3.2.0)-heptan-6-on, |
| 56 | 3-Hydroxy-2β-(3-phenyl-2-methyl-2-hydroxy-propylthio)-1,5-bicyclo(3.2.0)heptan-6-on, |
| 57 | 3-Hydroxy-2β-(3-(4-fluorphenyl)-2-methyl-2-hydroxy-propylthio)-1,5-bicyclo(3.2.0)-heptan-6-on, |
| 58 | 3-Hydroxy-2β-(3-(4-chlorphenyl)-2-methyl-2-hydroxy-propylthio)-1,5-bicyclo(3.2.0)-heptan-6-on, |
| 59 | 3-Hydroxy-2β-(3-(4-bromphenyl)-2-methyl-2-hydroxy-propylthio)-1,5-bicyclo(3.2.0)-heptan-6-on, |
| 60 | 3-Hydroxy-2β-(3-(4-methoxyphenyl)-2-methyl-2-hydroxy-propylthio)-1,5-bicyclo(3.2.0)-heptan-6-on, |
| 61 | 3-Hydroxy-2β-(3-(4-tolyl)-2-methyl-2-hydroxy-propylthio)-1,5-bicyclo(3.2.0)heptan-6-on, |
| 62 | 3-Hydroxy-2β-(3-(4-trifluormethylphenyl)-2-methyl-2-hydroxy-propylthio)-1,5-bicyclo-(3.2.0)heptan-6-on, |
| 63 | 3-Hydroxy-2β-(3-(3-chlorphenyl)-2-methyl-2-hydroxy-propylthio)-1,5-bicyclo(3.2.0)-heptan-6-on, |
| 64 | 3-Hydroxy-2β-(3-(3-trifluormethylphenyl)-2-methyl-2-hydroxy-propylthio)-1,5-bicyclo-(3.2.0)heptan-6-on, |
| 65 | 3-Hydroxy-2β-(4-phenyl-2-hydroxy-butylthio)-1,5-bicyclo(3.2.0)heptan-6-on, |
| 66 | 3-Hydroxy-2β-(4-(3-chlorphenyl)-2-hydroxy-butylthio)-1,5-bicyclo(3.2.0)-heptan-6-on, |
| 67 | 3-Hydroxy-2β-(4-(3-trifluormethylphenyl)-2-hydroxy-butylthio)-1,5-bicyclo(3.2.0)-heptan-6-on, |

Fortsetzung

| Beispiel | Verbindung der Formel I |
|---|---|
| 68 | 3-Hydroxy-2β-(4-phenyl-2-methyl-2-hydroxy-butylthio)-1,5-bicyclo(3.2.0)heptan-6-on, |
| 69 | 3-Hydroxy-2β-(4-(3-chlorphenyl)-2-methyl-2-hydroxy-butylthio)-1,5-bicyclo(3.2.0)-heptan-6-on, |
| 70 | 3-Hydroxy-2β-(4-(3-trifluormethylphenyl)-2-methyl-2-hydroxy-butylthio)-1,5-bicyclo-(3.2.0)heptan-6-on, |
| 71 | 3-Hydroxy-2β-(2-(2-naphthyl)-2-hydroxy-propylthio)-1,5-bicyclo(3.2.0)heptan-6-on, |
| 72 | 3-Hydroxy-2β-(2-(4-pyridyl)-2-hydroxy-propylthio)-1,5-bicyclo(3.2.0)heptan-6-on, |
| 73 | 3-Hydroxy-2β-(2-(2-thienyl)-2-hydroxy-propylthio)-1,5-bicyclo(3.2.0)heptan-6-on. |

Beispiel 74

Man löst 3,41 g 3α-Hydroxy-2β-(3-(3-chlorphenoxy)-2-hydroxypropylthio)-1βH,5βH-bicyclo(3.2.0)-heptan-6-on und 4 g Dihydropyran in 10 ml Methyl-tert.butyläther, versetzt mit 0,01 g wasserfreier p-Toluolsulfonsäure und rührt die Lösung 1 Stunde. Anschließend wäscht man mit Natriumbicarbonat-Lösung, trocknet über Natriumsulfat und destilliert das Lösungsmittel am Rotationsverdampfer ab. Man erhält 3,44 g 3α-Tetrahydropyranyl-(2)-oxy-2β-(3-(3-chlorphenoxy-2-tetrahydropyranyl-(2)-oxypropylthio)-1βH,5βH-bicyclo(3.2.0)heptan-6-on.

Beispiel 75

Man löst 3,41 g 3α-Hydroxy-2β-(3-(3-chlorphenoxy)-2-hydroxypropylthio)-1βH,5βH-bicyclo(3.2.0)-heptan-6-on zusammen mit 0,37 g Dimethylaminopyridin, 4,4 g Triäthylamin und 3 g Trimethylsilylchlorid unter Stickstoff in 20 ml Methylenchlorid und rührt die Lösung 2 Tage bei Raumtemperatur. Anschließend filtriert man den Niederschlag ab, wäscht das Filtrat zweimal mit gesättigter Ammoniumchlorid-Lösung, trocknet es über Natriumsulfat und destilliert das Lösungsmittel am Rotationsverdämpfer im Wasserstrahlvakuum ab. Man erhält 3,62 g 3α-Trimethylsilyloxy-2β-(3-(3-chlorphenoxy)-2-trimethylsilyloxy-propylthio)-1βH,5βH-bicyclo(3.2.0)heptan-6-on.

Beispiel 76

Man löst 20 g 3α-Hydroxy-2β-(3-(3-chlorphenoxy)-2-hydroxy-propylthio)-1βH,5βH-bicyclo(3.2.0)-heptan-6-on und 1 g Ammoniumacetat in 70 ml Methanol, leitet 30 Minuten lang Stickstoff durch die Lösung, fügt 1 ml 2,5-Dimethyl-2,4-hexadien hinzu und bestrahlt 2 Tage mit einer Hanau Q 600 Quecksilbermitteldrucklampe durch ein Pyrexfilter. Man destilliert das Lösungsmittel am Rotationsverdampfer ab, nimmt den Rückstand in Essigester auf und wäscht ihn mit 1 n Salzsäure und mit Wasser. Nach dem Trocknen über Natriumsulfat entfernt man das Lösungsmittel und löst den Rückstand in einer Mischung aus 50 ml Acetonitril und 25 ml 0,02 n Salzsäure. Man läßt 2 Tage stehen, macht mit gesättigter Sodalösung alkalisch, destilliert das Acetonitril am Rotationsverdampfer ab und extrahiert den wäßrigen Rückstand zweimal mit Essigester. Man trocknet die organischen Phasen über Natriumsulfat, gibt etwas Natriumbicarbonat zu und destilliert das Lösungsmittel ab. Anschließend chromatographiert man den Rückstand über Kieselgel und erhält 8,2 g 2-Oxa-3-hydroxy-6β-(3-(3-chlorphenoxy)-2-hydroxy-propylthio)-7α-hydroxy-1βH,5βH-bicyclo(3.2.0)octan.

IR: 1600, 1740, 2900 und 3400 $cm^{-1}$;
NMR: 0,9–1,4; 2,0–2,3; 2,8–3,1; 3,9–4,3; 6,7–7,3.

Analog Beispiel 76 sind aus den in den Beispielen 2 bis 75 genannten Verbindungen der Formel I durch Bestrahlen die in den folgenden Beispielen 77 bis 150 genannten Verbindungen der Formel IV erhältlich:

| Beispiel | Verbindung der Formel IV |
|---|---|
| 77 | 2-Oxa-3-hydroxy-6β-(3-phenoxy-2-hydroxy-propylthio)-7-hydroxy-1,5-bicyclo(3.3.0)-octan, |
| 78 | 2-Oxa-3-hydroxy-6β-(3-(4-fluorphenoxy)-2-hydroxy-propylthio)-7-hydroxy-1,5-bicyclo-(3.3.0)octan, |
| 79 | 2-Oxa-3-hydroxy-6β-(3-(4-chlorphenoxy)-2-hydroxy-propylthio)-7-hydroxy-1,5-bicyclo-(3.3.0)octan, |
| 80 | 2-Oxa-3-hydroxy-6β-(3-(4-bromphenoxy)-2-hydroxy-propylthio)-7-hydroxy-1,6-bicyclo-(3.3.0)octan, |
| 81 | 2-Oxa-3-hydroxy-6β-(3-(4-hydroxyphenoxy)-2-hydroxy-propylthio)-7-hydroxy-1,6-bicyclo(3.3.0)octan, |
| 82 | 2-Oxa-3-hydroxy-6β-(3-(4-methoxyphenoxy)-2-hydroxy-propylthio)-7-hydroxy-1,6-bicyclo(3.3.0)octan, |
| 83 | 2-Oxa-3-hydroxy-6β-(3-(4-tolyloxy)-2-hydroxy-propylthio)-7-hydroxy-1,6-bicyclo-(3.3.0)octan, |
| 84 | 2-Oxa-3-hydroxy-6β-(3-(4-trifluormethylphenoxy)-2-hydroxy-propylthio)-7-hydroxy-1,6-bicyclo(3.3.0)octan, |
| 85 | 2-Oxa-3-hydroxy-6β-(3-(3-methoxypehnoxy)-2-hydroxy-propylthio)-7-hydroxy-1,5-bicyclo(3.3.0)octan, |
| 86 | 2-Oxa-3-hydroxy-6β-(3-(3-trifluormethylphenoxy)-2-hydroxy-propylthio)-7-hydroxy-1,5-bicyclo(3.3.0)octan, |
| 87 | 2-Oxa-3-hydroxy-6β-(3-(2,4-dichlorphenoxy)-2-hydroxy-propylthio)-7-hydroxy-1,5-bicyclo(3.3.0)octan, |
| 88 | 2-Oxa-3-hydroxy-6β-(3-(2,4-dimethoxyphenoxy)-2-hydroxy-propylthio)-7-hydroxy-1,5-bicyclo(3.3.0)octan, |
| 89 | 2-Oxa-3-hydroxy-6β-(3-(2,4,6-trimethylphenoxy)-2-hydroxy-propylthio)-7-hydroxy-1,5-bicyclo(3.3.0)octan, |
| 90 | 2-Oxa-3-hydroxy-6β-(3-(3,4,5-trimethoxyphenoxy)-2-hydroxy-propylthio)-7-hydroxy-1,5-bicyclo(3.3.0)octan, |
| 91 | 2-Oxa-3-hydroxy-6β-(3-phenoxy-2-hydroxy-2-methyl-propylthio)-7-hydroxy-1,5-bicyclo(3.3.0)octan, |
| 92 | 2-Oxa-3-hydroxy-6β-(3-(4-fluorphenoxy)-2-hydroxy-2-methyl-propylthio)-7-hydroxy-1,5-bicyclo(3.3.0)octan, |
| 93 | 2-Oxa-3-hydroxy-6β-(3-(4-chlorphenoxy)-2-hydroxy-2-methyl-propylthio)-7-hydroxy-1,5-bicyclo(3.3.0)octan, |
| 94 | 2-Oxa-3-hydroxy-6β-(3-(4-bromphenoxy)-2-hydroxy-2-methyl-propylthio)-7-hydroxy-1,5-bicyclo(3.3.0)octan, |
| 95 | 2-Oxa-3-hydroxy-6β-(3-(4-hydroxyphenoxy)-2-hydroxy-2-methyl-propylthio)-7-hydroxy-1,5-bicyclo(3.3.0)octan, |
| 96 | 2-Oxa-3-hydroxy-6β-(3-(4-methoxyphenoxy)-2-hydroxy-2-methyl-propylthio)-7-hydroxy-1,5-bicyclo(3.3.0)octan, |
| 97 | 2-Oxa-3-hydroxy-6β-(3-(4-tolyloxy)-2-hydroxy-2-methyl-propylthio)-7-hydroxy-1,5-bicyclo(3.3.0)octan, |

Fortsetzung

| Beispiel | Verbindung der Formel IV |
|---|---|
| 98 | 2-Oxa-3-hydroxy-6β-(3-(4-trifluormethylphenoxy)-2-hydroxy-2-methyl-propylthio)-7-hydroxy-1,5-bicyclo(3.3.0)octan, |
| 99 | 2-Oxa-3-hydroxy-6β-(3-(3-chlorphenoxy)-2-hydroxy-2-methyl-propylthio)-7-hydroxy-1,5-bicyclo(3.3.0)octan, |
| 100 | 2-Oxa-3-hydroxy-6β-(3-(3-methoxyphenoxy)-2-hydroxy-2-methyl-propylthio)-7-hydroxy-1,5-bicyclo(3.3.0)octan, |
| 101 | 2-Oxa-3-hydroxy-6β-(3-(3-trifluormethylphenoxy)-2-hydroxy-2-methyl-propylthio)-7-hydroxy-1,5-bicyclo(3.3.0)octan, |
| 102 | 2-Oxa-3-hydroxy-6β-(3-(2,4-dichlorphenoxy)-2-hydroxy-2-methyl-propylthio)-7-hydroxy-1,5-bicyclo(3.3.0)octan, |
| 103 | 2-Oxa-3-hydroxy-6β-(3-(2,4-dimethoxyphenoxy)-2-hydroxy-2-methyl-propylthio)-7-hydroxy-1,5-bicyclo(3.3.0)octan, |
| 104 | 2-Oxa-3-hydroxy-6β-(3-(2,4,6-trimethylphenoxy)-2-hydroxy-2-methyl-propylthio)-7-hydroxy-1,5-bicyclo(3.3.0)octan, |
| 105 | 2-Oxa-3-hydroxy-6β-(3-(3,4,5-trimethoxyphenoxy)-2-hydroxy-2-methyl-propylthio)-7-hydroxy-1,5-bicyclo(3.3.0)octan, |
| 106 | 2-Oxa-3-hydroxy-6β-(2-hydroxy-2-methyl-heptylthio)-7-hydroxy-1,5-bicyclo(3.3.0)-octan, |
| 107 | 2-Oxa-3-hydroxy-6β-(2-hydroxy-heptylthio)-7-hydroxy-1,5-bicyclo(3.3.0)octan, |
| 108 | 2-Oxa-3-hydroxy-6β-(2-hydroxy-3-methyl-heptylthio)-7-hydroxy-1,5-bicyclo(3.3.0)-octan, |
| 109 | 2-Oxa-3-hydroxy-6β-(2-hydroxy-2,3-dimethyl-heptylthio)-7-hydroxy-1,5-bicyclo(3.3.0)-octan, |
| 110 | 2-Oxa-3-hydroxy-6β-(2-hydroxy-3,3-dimethyl-heptylthio)-7-hydroxy-1,5-bicyclo(3.3.0)-octan, |
| 111 | 2-Oxa-3-hydroxy-6β-(2-hydroxy-3-äthyl-heptylthio)-7-hydroxy-1,5-bicyclo(3.3.0)octan, |
| 112 | 2-Oxa-3-hydroxy-6β-(2-hydroxy-2-methyl-3-äthyl-heptylthio)-7-hydroxy-1,5-bicyclo-(3.3.0)octan, |
| 113 | 2-Oxa-3-hydroxy-6β-(2-hydroxy-4-äthyl-heptylthio)-7-hydroxy-1,5-bicyclo(3.3.0)octan, |
| 114 | 2-Oxa-3-hydroxy-6β-(2-hydroxy-2-methyl-4-äthyl-heptylthio)-7-hydroxy-1,5-bicyclo-(3.3.0)octan, |
| 115 | 2-Oxa-3-hydroxy-6β-(2-hydroxy-2-methyl-5-methoxy-pentylthio)-7-hydroxy-1,5-bicyclo(3.3.0)octan, |
| 116 | 2-Oxa-3-hydroxy-6β-(2-hydroxy-2-methyl-4-äthoxy-butylthio)-7-hydroxy-1,5-bicyclo(3.3.0)octan, |
| 117 | 2-Oxa-3-hydroxy-6β-(2-hydroxy-2-methyl-3-propyloxy-propylthio)-7-hydroxy-1,5-bicyclo(3.3.0)octan, |
| 118 | 2-Oxa-3-hydroxy-6β-(2-hydroxy-2-methyl-3-butyloxy-propylthio)-7-hydroxy-1,5-bicyclo(3.3.0)octan, |

Fortsetzung

| Beispiel | Verbindung der Formel IV |
|---|---|
| 119 | 2-Oxa-3-hydroxy-6β-(2-phenyl-2-hydroxy-propylthio)-7-hydroxy-1,5-bicyclo(3.3.0)octan, |
| 120 | 2-Oxa-3-hydroxy-6β-(2-(4-fluorphenyl)-2-hydroxy-propylthio)-7-hydroxy-1,5-bicyclo(3.3.0)octan, |
| 121 | 2-Oxa-3-hydroxy-6β-(2-4-chlorphenyl)2-hydroxy-propylthio)-7-hydroxy-1,5-bicyclo(3.3.0)octan, |
| 122 | 2-Oxa-3-hydroxy-6β-(2-(4-bromphenyl)-2-hydroxy-propylthio)-7-hydroxy-1,5-bicyclo(3.3.0)octan, |
| 123 | 2-Oxa-3-hydroxy-6β-(2-(4-hydroxyphenyl)-2-hydroxy-propylthio)-7-hydroxy-1,5-bicyclo(3.3.0)octan, |
| 124 | 2-Oxa-3-hydroxy-6β-(2-(4-methoxyphenyl)-2-hydroxy-propylthio)-7-hydroxy-1,5-bicyclo(3.3.0)octan, |
| 125 | 2-Oxa-3-hydroxy-6β-(2-(4-tolyl)-2-hydroxy-propylthio)-7-hydroxy-1,5-bicyclo(3.3.0)octan, |
| 126 | 2-Oxa-3-hydroxy-6β-(2-(4-trifluormethylphenyl)-2-hydroxy-propylthio)-7-hydroxy-1,5-bicyclo(3.3.0)octan, |
| 127 | 2-Oxa-3-hydroxy-6β-(2-(3-methoxyphenyl)-2-hydroxy-propylthio)-7-hydroxy-1,5-bicyclo(3.3.0)octan, |
| 128 | 2-Oxa-3-hydroxy-6β-(2-(3-chlorphenyl)-2-hydroxy-propylthio)-7-hydroxy-1,5-bicyclo(3.3.0)octan, |
| 129 | 2-Oxa-3-hydroxy-6β-(2-(3-trifluormethylphenyl)-2-hydroxy-propylthio)-7-hydroxy-1,5-bicyclo(3.3.0)octan, |
| 130 | 2-Oxa-3-hydroxy-6β-(2-(3,4,5-trimethoxyphenyl)-2-hydroxy-propylthio)-7-hydroxy-1,5-bicyclo(3.3.0)octan, |
| 131 | 2-Oxa-3-hydroxy-6β-(3-phenyl-2-methyl-2-hydroxy-propylthio)-7-hydroxy-1,5-bicyclo(3.3.0)octan, |
| 132 | 2-Oxa-3-hydroxy-6β-(3-(4-fluorpenyl)-2-methyl-2-hydroxy-propylthio)-7-hydroxy-1,5-bicyclo(3.3.0)octan, |
| 133 | 2-Oxa-3-hydroxy-6β-(3-(4-chlorphenyl)-2-methyl-2-hydroxy-propylthio)-7-hydroxy-1,5-bicyclo(3.3.0)octan, |
| 134 | 2-Oxa-3-hydroxy-6β-(3-(4-bromphenyl)-2-methyl-2-hydroxy-propylthio)-7-hydroxy-1,5-bicyclo(3.3.0)octan, |
| 135 | 2-Oxa-3-hydroxy-6β-(3-(4-methoxyphenyl)-2-methyl-2-hydroxy-propylthio)-7-hydroxy-1,5-bicyclo(3.3.0)octan, |
| 136 | 2-Oxa-3-hydroxy-6β-(3-(4-tolyl)-2-methyl-2-hydroxy-propylthio)-7-hydroxy-1,5-bicyclo(3.3.0)octan, |
| 137 | 2-Oxa-3-hydroxy-6β-(3-(4-trifluormethylphenyl)-2-methyl-2-hydroxy-propylthio)-7-hydroxy-1,5-bicyclo(3.3.0)octan, |
| 138 | 2-Oxa-3-hydroxy-6β-(3-(3-chlorphenyl)-2-methyl-2-hydroxy-propylthio)-7-hydroxy-1,5-bicyclo(3.3.0)octan, |

Fortsetzung

| Beispiel | Verbindung der Formel IV |
|---|---|
| 139 | 2-Oxa-3-hydroxy-6β-(3-(3-trifluormethylphenyl)-2-methyl-2-hydroxy-propylthio)-7-hydroxy-1,5-bicyclo(3.3.0)octan, |
| 140 | 2-Oxa-3-hydroxy-6β-(4-phenyl-2-hydroxy-butylthio)-7-hydroxy-1,5-bicyclo(3.3.0)octan, |
| 141 | 2-Oxa-3-hydroxy-6β-(4-(3-chlorphenyl)-2-hydroxy-butylthio)-7-hydroxy-1,5-bicyclo(3.3.0)octan, |
| 142 | 2-Oxa-3-hydroxy-6β-(4-(3-trifluormethylphenyl)-2-hydroxy-butylthio)-7-hydroxy-1,5-bicyclo(3.3.0)octan, |
| 143 | 2-Oxa-3-hydroxy-6β-(4-phenyl-2-methyl-2-hydroxy-butylthio)-7-hydroxy-1,5-bicyclo(3.3.0)octan, |
| 144 | 2-Oxa-3-hydroxy-6β-(4-(3-chlorphenyl)-2-methyl-2-hydroxy-butylthio)-7-hydroxy-1,5-bicyclo(3.3.0)octan, |
| 145 | 2-Oxa-3-hydroxy-6β-(4-(3-trifluormethylphenyl-2-methyl-2-hydroxy-butylthio)-7-hydroxy-1,5-bicyclo(3.3.0)octan, |
| 146 | 2-Oxa-3-hydroxy-6β-(2-(2-naphthyl)-2-hydroxy-propylthio)-7-hydroxy-1,5-bicyclo(3.3.0)octan, |
| 147 | 2-Oxa-3-hydroxy-6β-(2-(4-pyridyl)-2-hydroxy-propylthio)-7-hydroxy-1,5-bicyclo(3.3.0)octan, |
| 148 | 2-Oxa-3-hydroxy-6β-(2-(2-thienyl)-2-hydroxy-propylthio)-7-hydroxy-1,5-bicyclo(3.3.0)octan, |
| 149 | 2-Oxa-3-hydroxy-6β-(3-(3-chlorphenoxy)-2-tetrahydropyranyl-(2)-oxy-propylthio)-7α-tetrahydropyranyl-(2)-oxy-1βH, 5βH-bicyclo(3.3.0)octan, |
| 150 | 2-Oxa-3-hydroxy-6β-(3-(3-chlorphenoxy)-2-trimethyl-silyloxy-propylthio)-7α-trimethylsilyloxy-1βH, 5βH-bicyclo(3.3.0)octan. |

## Beispiel 151

Man löst 8 g 2-Oxa-3-hydroxy-6β-(3-(3-chlorphenoxy)-2-hydroxy-propylthio)-7α-hydroxy-1βH,5βH-bicyclo(3.2.0)octan in 50 ml Tetrahydrofuran (THF) und tropft diese Lösung unter Kühlung zu einer Lösung von 12,6 g Kaliumtertiärbutylat und 14,8 g Carboxybutyltriphenylphosphoniumbromid in 50 ml THF. Man rührt noch 1 Stunde nach, läßt anschließend 50 ml Wasser zutropfen und destilliert das THF am Rotationsverdampfer ab. Die resultierende wäßrige Lösung wird dreimal mit Äthylacetat gewaschen und mit Zitronensäure bis pH 3 angesäuert. Man extrahiert dreimal mit Äthylacetat, trocknet die Lösung über Natriumsulfat und destilliert das Lösungsmittel am Rotationsverdampfer ab. Der Rückstand wird in 200 ml Diäthyläther aufgenommen und über Nacht bei 0°C aufbewahrt. Man filtriert, destilliert das Lösungsmittel ab und erhält nach chromatographischer Reinigung über 50 g Kieselgel 7,0 g 9α,11α,15-Trihydroxy-16-(3-chlorphenoxy)-13-thia-17,18,19,20-tetranor-(Z)-5-prostensäure.

IR: 1710, 2400, 3650 $cm^{-1}$;
NMR: 1,1–2,6; 2,6–3,1; 4,05; 4,2; 5,3; 5,4; 6,6–7,4.

Analog Beispiel 151 sind aus den in den Beispielen 77 bis 148 genannten Verbindungen der Formel IV durch Umsetzen mit Carboxybutyltriphenylphosphoniumbromid in Gegenwart von Kaliumtertiärbutylat die in den folgenden Beispielen 152 bis 223 genannten Verbindungen der Formel III erhältlich:

| Beispiel | Verbindung der Formel III mit D = –CHOH– und E = –CH=CH– |
|---|---|
| 152 | 9,11,15-Trihydroxy-16-phenoxy-13-thia-17,18,19,20-tetranor-5-prostensäure, |
| 153 | 9,11,15-Trihydroxy-16-(4-fluorphenoxy)-13-thia-17,18,19,20-tetranor-5-prostensäure,<br>IR: 1710, 2700, 2940, 3700 $cm^{-1}$;<br>NMR: 1,1–2,6; 2,7–3,1; 4,05; 4,2; 5,3; 5,45; 6,85; 6,95; |
| 154 | 9,11,15-Trihydroxy-16-(4-chlorphenoxy)-13-thia-17,18,19,20-tetranor-5-prostensäure, |
| 155 | 9,11,15-Trihydroxy-16-(4-bromphenoxy)-13-thia-17,18,19,20-tetranor-5-prostensäure, |
| 156 | 9,11,15-Trihydroxy-16-(4-hydroxyphenoxy)-13-thia-17,18,19,20-tetranor-5-prostensäure, |
| 157 | 9,11,15-Trihydroxy-16-(4-methoxyphenoxy)-13-thia-17,18,19,20-tetranor-5-prostensäure, |
| 158 | 9,11,15-Trihydroxy-16-(4-tolyloxy)-13-thia-17,18,19,20-tetranor-5-prostensäure, |
| 159 | 9,11,15-Trihydroxy-16-(4-trifluormethylphenoxy)-13-thia-17,18,19,20-tetranor-5-prostensäure, |
| 160 | 9,11,15-Trihydroxy-16-(3-methoxyphenoxy)-13-thia-17,18,19,20-tetranor-5-prostensäure, |
| 161 | 9,11,15-Trihydroxy-16-(3-trifluormethylphenoxy)-13-thia-17,18,19,20-tetranor-5-prostensäure, |
| 162 | 9,11,15-Trihydroxy-16-(2,4-dichlorphenoxy)-13-thia-17,18,19,20-tetranor-5-prostensäure, |
| 163 | 9,11,15-Trihydroxy-16-(2,4-dimethoxyphenoxy)-13-thia-17,18,19,20-tetranor-5-prostensäure, |
| 164 | 9,11,15-Trihydroxy-16-(2,4,6-trimethylphenoxy)-13-thia-17,18,19,20-tetranor-5-prostensäure, |
| 165 | 9,11,15-Trihydroxy-16-(3,4,5-trimethoxyphenoxy)-13-thia-17,18,19,20-tetranor-5-prostensäure, |
| 166 | 9,11,15-Trihydroxy-15-methyl-16-phenoxy-13-thia-17,18,19,20-tetranor-5-prostensäure, |
| 167 | 9,11,15-Trihydroxy-15-methyl-16-(4-fluorphenoxy)-13-thia-17,18,19,20-tetranor-5-prostensäure, |
| 168 | 9,11,15-Trihydroxy-15-methyl-16-(4-chlorphenoxy)-13-thia-17,18,19,20-tetranor-5-prostensäure, |
| 169 | 9,11,15-Trihydroxy-15-methyl-16-(4-bromphenoxy)-13-thia-17,18,19,20-tetranor-5-prostensäure, |
| 170 | 9,11,15-Trihydroxy-15-methyl-16-(4-hydroxyphenoxy)-13-thia-17,18,19,20-tetranor-5-prostensäure, |
| 171 | 9,11,15-Trihydroxy-15-methyl-16-(4-methoxyphenoxy)-13-thia-17,18,19,20-tetranor-5-prostensäure, |
| 172 | 9,11,15-Trihydroxy-15-methyl-16-(4-tolyloxy)-13-thia-17,18,19,20-tetranor-5-prostensäure, |

Fortsetzung

| Beispiel | Verbindung der Formel III mit D = —CHOH— und E = —CH=CH— |
|---|---|
| 173 | 9,11,15-Trihydroxy-15-methyl-16-(4-trifluormethylphenoxy)-13-thia-17,18,19,20-tetranor-5-prostensäure, |
| 174 | 9,11,15-Trihydroxy-15-methyl-16-(3-chlorphenoxy)-13-thia-17,18,19,20-tetranor-5-prostensäure, |
| | IR: 1600, 1710, 2900, 3400 $cm^{-1}$; |
| | NMR: 1,4; 4,20; 4,95; 5,45; 6,7—7,3; |
| 175 | 9,11,15-Trihydroxy-15-methyl-16-(3-methoxyphenoxy)-13-thia-17,18,19,20-tetranor-5-prostensäure, |
| 176 | 9,11,15-Trihydroxy-15-methyl-16-(3-trifluormethylphenoxy)-13-thia-17,18,19,20-tetranor-5-prostensäure, |
| 177 | 9,11,15-Trihydroxy-15-methyl-16-(2,4-dichlorphenoxy)-13-thia-17,18,19,20-tetranor-5-prostensäure, |
| 178 | 9,11,15-Trihydroxy-15-methyl-16-(2,4-dimethoxyphenoxy)-13-thia-17,18,19,20-tetranor-5-prostensäure, |
| 179 | 9,11,15-Trihydroxy-15-methyl-16-(2,4,6-trimethylphenoxy)-13-thia-17,18,19,20-tetranor-5-prostensäure, |
| 180 | 9,11,15-Trihydroxy-15-methyl-16-(3,4,5-trimethoxyphenoxy)-13-thia-17,18,19,20-tetranor-5-prostensäure, |
| 181 | 9,11,15-Trihydroxy-15-methyl-13-thia-5-prostensäure, |
| | IR: 1710, 2200, 3700 $cm^{-1}$; |
| | NMR: 0,85; 1,2; 1,0—2,6; 2,6—3,5; 4,15; 5,1—5,4; |
| 182 | 9,11,15-Trihydroxy-13-thia-5-prostensäure, |
| 183 | 9,11,15-Trihydroxy-16-methyl-13-thia-5-prostensäure, |
| 184 | 9,11,15-Trihydroxy-15,16-dimethyl-13-thia-5-prostensäure, |
| 185 | 9,11,15-Trihydroxy-16,16-dimethyl-13-thia-5-prostensäure, |
| 186 | 9,11,15-Trihydroxy-16-äthyl-13-thia-5-prostensäure, |
| 187 | 9,11,15-Trihydroxy-15-methyl-16-äthyl-13-thia-5-prostensäure, |
| 188 | 9,11,15-Trihydroxy-17-äthyl-13-thia-5-prostensäure, |
| 189 | 9,11,15-Trihydroxy-15-methyl-17-äthyl-13-thia-5-prostensäure, |
| 190 | 9,11,15-Trihydroxy-15-methyl-13-thia-19-oxa-5-prostensäure, |
| 191 | 9,11,15-Trihydroxy-15-methyl-13-thia-18-oxa-5-prostensäure, |
| 192 | 9,11,15-Trihydroxy-15-methyl-13-thia-17-oxa-5-prostensäure, |
| 193 | 9,11,15-Trihydroxy-15-methyl-13-thia-17-oxa-20-homo-5-prostensäure, |
| 194 | 9,11,15-Trihydroxy-15-phenyl-13-thia-17,18,19,20-tetranor-5-prostensäure, |

Fortsetzung

| Beispiel | Verbindung der Formel III mit D = –CHOH– und E = –CH=CH– |
|---|---|
| 195 | 9,11,15-Trihydroxy-15-(4-fluorphenyl)-13-thia-17,18,19,20-tetranor-5-prostensäure, |
| 196 | 9,11,15-Trihydroxy-15-(4-chlorphenyl)-13-thia-17,18,19,20-tetranor-5-prostensäure, |
| 197 | 9,11,15-Trihydroxy-15-(4-bromphenyl)-13-thia-17,18,19,20-tetranor-5-prostensäure, |
| 198 | 9,11,15-Trihydroxy-15-(4-hydroxyphenyl)-13-thia-17,18,19,20-tetranor-5-prostensäure, |
| 199 | 9,11,15-Trihydroxy-15-(4-methoxyphenyl)-13-thia-17,18,19,20-tetranor-5-prostensäure, |
| 200 | 9,11,15-Trihydroxy-15-(4-tolyl)-13-thia-17,18,19,20-tetranor-5-prostensäure, |
| 201 | 9,11,15-Trihydroxy-15-(4-trifluormethylphenyl)-13-thia-17,18,19,20-tetranor-5-prostensäure, |
| 202 | 9,11,15-Trihydroxy-15-(3-methoxyphenyl)-13-thia-17,18,19,20-tetranor-5-prostensäure, |
| 203 | 9,11,15-Trihydroxy-15-(3-chlorphenyl)-13-thia-17,18,19,20-tetranor-5-prostensäure, |
| 204 | 9,11,15-Trihydroxy-15-(3-trifluormethylphenyl)-13-thia-17,18,19,20-tetranor-5-prostensäure, |
| 205 | 9,11,15-Trihydroxy-15-(3,4,5-trimethoxyphenyl)-13-thia-17,18,19,20-tetranor-5-prostensäure, |
| 206 | 9,11,15-Trihydroxy-15-methyl-16-phenyl-13-thia-17,18,19,20-tetranor-5-prostensäure, |
| 207 | 9,11,15-Trihydroxy-15-methyl-16-(4-fluorphenyl)-13-thia-17,18,19,20-tetranor-5-prostensäure, |
| 208 | 9,11,15-Trihydroxy-15-methyl-16-(4-chlorphenyl)-13-thia-17,18,19,20-tetranor-5-prostensäure, |
| 209 | 9,11,15-Trihydroxy-15-methyl-16-(4-bromphenyl)-13-thia-17,18,19,20-tetranor-5-prostensäure, |
| 210 | 9,11,15-Trihydroxy-15-methyl-16-(4-methoxyphenyl)-13-thia-17,18,19,20-tetranor-5-prostensäure, |
| 211 | 9,11,15-Trihydroxy-15-methyl-16-(4-tolyl)-13-thia-17,18,19,20-tetranor-5-prostensäure, |
| 212 | 9,11,15-Trihydroxy-15-methyl-16-(4-trifluormethylphenyl)-13-thia-17,18,19,20-tetranor-5-prostensäure, |
| 213 | 9,11,15-Trihydroxy-15-methyl-16-(3-chlorphenyl)-13-thia-17,18,19,20-tetranor-5-prostensäure, |
| 214 | 9,11,15-Trihydroxy-15-methyl-16-(3-trifluormethylphenyl)-13-thia-17,18,19,20-tetranor-5-prostensäure, |
| 215 | 9,11,15-Trihydroxy-17-phenyl-13-thia-18,19,20-trinor-5-prostensäure, |

Fortsetzung

| Beispiel | Verbindung der Formel III mit D = —CHOH— und E = —CH=CH— |
|---|---|
| 216 | 9,11,15-Trihydroxy-17-(3-chlorphenyl)-13-thia-18,19,20-trinor-5-prostensäure, |
| 217 | 9,11,15-Trihydroxy-17-(3-trifluormethylphenyl)-13-thia-18,19,20-trinor-5-prostensäure, |
| 218 | 9,11,15-Trihydroxy-15-methyl-17-phenyl-13-thia-18,19,20-trinor-5-prostensäure, |
| | IR: 1600, 1700, 2950, 3400 $cm^{-1}$; |
| | NMR: 1,35; 4,18; 5,42; 7,22; |
| 219 | 9,11,15-Trihydroxy-15-methyl-17-(3-chlorphenyl)-13-thia-18,19,20-trinor-5-prostensäure, |
| | IR: 1050, 1240, 1600, 1710, 2900, 3400 $cm^{-1}$; |
| | NMR: 1,32; 4,0—4,3; 5,35; 7,0—7,2; |
| 220 | 9,11,15-Trihydroxy-15-methyl-17-(3-trifluormethylphenyl)-23-thia-18,19,20-trinor-5-prostensäure, |
| | IR: 1720, 3450 $cm^{-1}$; |
| | NMR: 1,35; 5,25; 5,48; 7,45; |
| 221 | 9,11,15-Trihydroxy-15-(2-naphthyl)-13-thia-17,18,19,20-tetranor-5-prostensäure, |
| 222 | 9,11,15-Trihydroxy-15-(4-pyridyl)-13-thia-17,18,19,20-tetranor-5-prostensäure, |
| 223 | 9,11,15-Trihydroxy-15-(2-thienyl)-13-thia-17,18,19,20-tetranor-5-prostensäure. |

## Beispiel 224

Man tropft unter Stickstoff eine Lösung von 5,6 g 4-(4-Benzamido)phenyloxycarbonylbutyltriphenylphosphoniumbromid, gelöst in 15 ml trockenem THF zu einer gerührten Lösung, welche durch Zugabe von 2 g Kalium-tert.butylat zu 10 ml trockenem THF erhalten wurde. Anschließend tropft man unter Stickstoff und Rühren 3 g 2-Oxa-3,7-dihydroxy-6β-(2-hydroxy-2-methylheptylthio)-1,5-bicyclo[3,3,0]octan, gelöst in 5 ml trockenem THF, zur Lösung des Phosphorylids und rührt 1 Stunde bei Raumtemperatur. Danach gießt man das Reaktionsgemisch in ein Gemisch, bestehend aus 10 ml Äthylacetat, 40 g Trockeneis und 50 ml Wasser, trennt die organische Phase ab, wäscht die wässerige Phase dreimal mit je 50 ml Äthylacetat, enthaltend 20 g Trockeneis, wäscht die vereinigten organischen Phasen mit Wasser, trocknet über $MgSO_4$, destilliert das Lösungsmittel ab und erhält nach chromatographischer Reinigung des Rückstandes (Kieselgel/Chloroform) 9,11,15-Trihydroxy-15-methyl-13-thia-5-prostensäure-p-benzoylaminophenylester.

## Beispiel 225

Man löst 1 g 9α,11α,15-Trihydroxy-16-(3-chlorphenoxy)-13-thia-17,18,19,20-tetranor-(Z)-5-prostensäure in 10 ml Essigsäureäthylester, leitet 10 Minuten lang Wasserstoff durch die Lösung, versetzt anschließend mit 0,1 g Palladium auf Kohle und hydriert mit 1 atm Wasserstoff bis zur berechneten Wasserstoff-Aufnahme. Nach beendeter Reaktion filtriert man den Katalysator ab und destilliert das Lösungsmittel ab. Man erhält ein gelbes Öl, das in Äther aufgenommen wird. Beim Abkühlen kristallisieren 0,8 g 9α,11α,15-Trihydroxy-16-(3-chlorphenoxy)-13-thia-17,18,19,20-tetranor-prostansäure aus.

IR: 1600, 1710, 2950, 3400 $cm^{-1}$;
NMR: 1,1–2,6; 2,7–3,1; 4,1; 4,2; 6,7–7,3.

Analog Beispiel 225 sind aus den in den Beispielen 152 bis 223 genannten Verbindungen der Formel III durch katalytische Hydrierung die entsprechenden 9,11,15-Trihydroxy-13-thiaprostansäurederivate erhältlich, insbesondere die in den folgenden Beispielen 226 bis 255 genannten Verbindungen:

| Beispiel | Verbindung der Formel III mit D = –CHOH– und E = $-CH_2{=}CH_2-$ |
|---|---|
| 226 | 9,11,15-Trihydroxy-16-phenoxy-13-thia-17,18,19,20-tetranor-prostansäure, |
| 227 | 9,11,15-Trihydroxy-16-(4-fluorphenoxy)-13-thia-17,18,19,20-tetranor-prostansäure, |
| 228 | 9,11,15-Trihydroxy-16-(4-chlorphenoxy)-13-thia-17,18,19,20-tetranor-prostansäure, |
| 229 | 9,11,15-Trihydroxy-16-(4-trifluormethylphenoxy)-13-thia-17,18,19,20-tetranor-prostansäure, |
| 230 | 9,11,15-Trihydroxy-16-(3-trifluormethylphenoxy)-13-thia-17,18,19,20-tetranor-prostansäure, |
| | IR: 1590, 1700, 2900, 3400 $cm^{-1}$; |
| | NMR: 1,1–2,5; 4,0; 4,2–4,4; 6,9–7,4; |
| 231 | 9,11,15-Trihydroxy-15-methyl-16-(4-fluorphenoxy)-13-thia-17,18,19,20-tetranor-prostansäure, |
| | IR: 1220, 1710, 3400 $cm^{-1}$; |
| | NMR: 1,37; 2,3; 3,85; 4,14; 6,7–7,0; |
| 232 | 9,11,15-Trihydroxy-15-methyl-16-(4-chlorphenoxy)-13-thia-17,18,19,20-tetranor-prostansäure, |
| 233 | 9,11,15-Trihydroxy-15-methyl-16-(4-trifluormethylphenoxy)-13-thia-17,18,19,20-tetranor-prostansäure, |
| 234 | 9,11,15-Trihydroxy-15-methyl-16-(3-chlorphenoxy)-13-thia-17,18,19,20-tetranor-prostansäure, |
| 235 | 9,11,15-Trihydroxy-15-methyl-16-(3-trifluormethylphenoxy)-13-thia-17,18,19,20-tetranor-prostansäure, |
| 236 | 9,11,15-Trihydroxy-15-methyl-13-thia-prostansäure, |
| | IR: 1705, 2680 und 3340 $cm^{-1}$; |
| | NMR: 0,89; 1,22; 2,3; 4,18; 5,03; |
| 237 | 9,11,15-Trihydroxy-13-thia-prostansäure, |
| 238 | 9,11,15-Trihydroxy-16-methyl-13-thia-prostansäure, |
| 239 | 9,11,15-Trihydroxy-15,16-dimethyl-13-thia-prostansäure, |
| 240 | 9,11,15-Trihydroxy-16,16-dimethyl-13-thia-prostansäure, |
| 241 | 9,11,15-Trihydroxy-15-phenyl-13-thia-17,18,19,20-tetranor-prostansäure, |
| 242 | 9,11,15-Trihydroxy-15-(4-fluorphenyl)-13-thia-17,18,19,20-tetranor-prostansäure, |
| 243 | 9,11,15-Trihydroxy-15-(4-chlorphenyl)-13-thia-17,18,19,20-tetranor-prostansäure, |

Fortsetzung

| Beispiel | Verbindung der Formel III mit D = $-CHOH-$ und E = $-CH_2{=}CH_2-$ |
|---|---|
| 244 | 9,11,15-Trihydroxy-15-(4-tolyl)-13-thia-17,18,19,20-tetranor-prostansäure, |
| | IR: 1505, 1705, 3350 $cm^{-1}$; |
| | NMR: 1,2; 2,3; 4,2; 4,6; 7,0–7,3; |
| 245 | 9,11,15-Trihydroxy-15-(3-chlorphenyl)-13-thia-17,18,19,20-tetranor-prostansäure, |
| 246 | 9,11,15-Trihydroxy-15-(3-trifluormethylphenyl)-13-thia-17,18,19,20-tetranor-prostansäure, |
| 247 | 9,11,15-Trihydroxy-15-methyl-16-phenyl-13-thia-17,18,19,20-tetranor-prostansäure, |
| 248 | 9,11,15-Trihydroxy-15-methyl-16-(4-fluorphenyl)-13-thia-17,18,19,20-tetranor-prostansäure, |
| 249 | 9,11,15-Trihydroxy-15-methyl-16-(4-tolyl)-13-thia-17,18,19,20-tetranor-prostansäure, |
| 250 | 9,11,15-Trihydroxy-17-phenyl-13-thia-18,19,20-trinor-prostansäure, |
| 251 | 9,11,15-Trihydroxy-17-(3-chlorphenyl)-13-thia-18,19,20-trinor-prostansäure, |
| 252 | 9,11,15-Trihydroxy-17-(3-trifluormethylphenyl)-13-thia-18,19,20-trinor-prostansäure, |
| 253 | 9,11,15-Trihydroxy-15-methyl-17-phenyl-13-thia-18,19,20-trinor-prostansäure, |
| | IR: 1700, 3400 $cm^{-1}$; |
| | NMR: 1,33; 4,4; 7,25; |
| 254 | 9,11,15-Trihydroxy-15-methyl-17-(3-chlorphenyl)-13-thia-18,19,20-trinor-prostansäure, |
| 255 | 9,11,15-Trihydroxy-15-methyl-17-(3-trifluormethylphenyl)-13-thia-18,19,20-trinor-prostansäure. |

## Beispiel 256

Man löst 0,44 g 9α,11α,15-Trihydroxy-16-(3-chlorphenoxy)-13-thia-17,18,19,20-tetranor-prostansäure, 0,22 g Triäthylamin, 0,037 g Dimethylaminopyridin und 0,33 g tert.-Butyldimethylsilylchlorid bei 0°C unter Stickstoff-Atmosphäre in 10 ml Methylenchlorid. Man läßt auf Raumtemperatur kommen und rührt über Nacht. Anschließend saugt man den Niederschlag ab, wäscht das Filtrat zweimal mit gesättigter Ammoniumchloridlösung, trocknet es über $MgSO_4$ und zieht das Lösungsmittel am Rotationsverdampfer ab.

Den Rückstand löst man in Aceton, tropft bei −20°C eine eiskalte Lösung von 0,15 ml 8 N Jones-Reagenz (Chromsäureanhydrid in verdünnter Schwefelsäure) in 5 ml Aceton zu, rührt eine Stunde nach und zerstört dann überschüssige Chromsäure mit 1 ml Isopropanol. Danach läßt man auf Raumtemperatur kommen, gibt 10 ml 10%ige Schwefelsäure zu und rührt noch 1 Stunde bei Raumtemperatur. Das Aceton wird am Rotationsverdampfer abgezogen, und die wäßrige Phase wird dreimal mit Methylenchlorid extrahiert. Man trocknet die vereinigten organischen Phasen über $MgSO_4$ und engt zum Rückstand ein. Der Rückstand wird über 50 g Kieselgel mit Äther chromatographiert. Man erhält 255 mg kristallines 11α,15-Dihydroxy-9-oxo-16-(3-chlorphenoxy)-13-thia-17,18,19,20-tetranor-prostansäure.

Analog Beispiel 256 sind aus Verbindungen der Formel III mit D = $-CHOH-$ und E = $-CH_2-CH_2-$ durch Oxidation die entsprechenden Verbindungen mit D = $-CO-$ erhältlich, insbesondere die in den folgenden Beispielen 257 bis 293 genannten Verbindungen:

| Beispiel | Verbindung der Formel III mit D = —CO— und E = $-CH_2CH_2-$ |
|---|---|
| 257 | 11,15-Dihydroxy-9-oxo-16-phenoxy-13-thia-17,18,19,20-tetranor-prostansäure, |
| 258 | 11,15-Dihydroxy-9-oxo-16-(4-fluorphenoxy)-13-thia-17,18,19,20-tetranor-prostansäure, |
| 259 | 11,15-Dihydroxy-9-oxo-16-(4-methoxyphenoxy)-13-thia-17,18,19,20-tetranor-prostansäure, |
| 260 | 11,15-Dihydroxy-9-oxo-16-(3-trifluormethylphenoxy)-13-thia-17,18,19,20-tetranor-prostansäure, |
| | IR: 1230, 1490, 1590, 1700, 1730, 2950, 3410 $cm^{-1}$; |
| | NMR: 2,3; 4,1; 4,2–4,5; 7,0–7,5; |
| 261 | 11,15-Dihydroxy-9-oxo-15-methyl-16-phenoxy-13-thia-17,18,19,20-tetranor-prostansäure, |
| 262 | 11,15-Dihydroxy-9-oxo-15-methyl-16-(4-fluorphenoxy)-13-thia-17,18,19,20-tetranor-prostansäure, |
| | IR: 1215, 1500, 1710, 1740, 3350 $cm^{-1}$; |
| | NMR: 1,4; 2,3; 3,9; 4,3; 6,8–7,1; |
| 263 | 11,15-Dihydroxy-9-oxo-15-methyl-16-(4-chlorphenoxy)-13-thia-17,18,19,20-tetranor-prostansäure, |
| 264 | 11,15-Dihydroxy-9-oxo-15-methyl-16-(4-methoxyphenoxy)-13-thia-17,18,19,20-tetranor-prostansäure, |
| 265 | 11,15-Dihydroxy-9-oxo-15-methyl-16-(3-trifluormethylphenoxy)-13-thia-17,18,19,20-tetranor-prostansäure, |
| | IR: 1230, 1480, 1590, 1710, 1740, 2900, 3400 $cm^{-1}$; |
| | NMR: 1,35; 2,25; 3,9; 4,3; 6,9–7,4; |
| 266 | 11,15-Dihydroxy-9-oxo-15-methyl-13-thia- prostansäure, |
| | IR: 1710, 1740, 3450 $cm^{-1}$; |
| | NMR: 0,85; 1,2; 2,3; 4,2; 5,8; |
| 267 | 11,15-Dihydroxy-9-oxo-13-thia-prostansäure, |
| 268 | 11,15-Dihydroxy-9-oxo-16-methyl-13-thia-prostansäure, |
| | IR: 1700, 1720, 3350 $cm^{-1}$; |
| | NMR: 0,94; 3,68; 4,27; 5,88; |
| 269 | 11,15-Dihydroxy-9-oxo-15,16-dimethyl-13-thia-prostansäure, |
| | IR: 1710, 1740, 3400 $cm^{-1}$; |
| | NMR: 0,88; 0,97; 1,2; 4,3; 6,0; |
| 270 | 11,15-Dihydroxy-9-oxo-16,16-dimethyl-13-thia-prostansäure, |

Fortsetzung

| Beispiel | Verbindung der Formel III mit D = $-CO-$ und E = $-CH_2CH_2-$ |
|---|---|
| 271 | 11,15-Dihydroxy-9-oxo-17-äthyl-13-thia-prostansäure,<br>IR: 1720, 1740, 3450 $cm^{-1}$;<br>NMR: 3,9; 4,29; 5,86; |
| 272 | 11,15-Dihydroxy-9-oxo-15-methyl-13-thia-19-oxa-prostansäure,<br>IR: 1710, 1740, 3400 $cm^{-1}$;<br>NMR: 1,27; 2,32; 3,31; 422; |
| 273 | 11,15-Dihydroxy-9-oxo-15-methyl-13-thia-18-oxa-prostansäure,<br>IR: 1715, 1740, 3400 $cm^{-1}$;<br>NMR: 1,21; 1,32; 2,31; 3,50; 4,28; |
| 274 | 11,15-Dihydroxy-9-oxo-15-phenyl-13-thia-16,17,18,19,20-pentanor-prostansäure, |
| 275 | 11,15-Dihydroxy-9-oxo-15-(4-tolyl)-13-thia-16,17,18,19,20-pentanor-prostansäure,<br>IR: 1710, 1740, 3400 $cm^{-1}$;<br>NMR: 2,3; 4,21; 7,15–7,4; |
| 276 | 11,15-Dihydroxy-9-oxo-15-phenyl-13-thia-17,18,19,20-tetranor-prostansäure, |
| 277 | 11,15-Dihydroxy-9-oxo-15-(4-fluorphenyl)-13-thia-17,18,19,20-tetranor-prostansäure,<br>IR: 1220, 1505, 1605, 1708, 1740, 3400, $cm^{-1}$;<br>NMR: 1,61; 2,3; 4,2; 6,9–7,15; 7,3–7,5; |
| 278 | 11,15-Dihydroxy-9-oxo-15-(4-chlorphenyl)-13-thia-17,18,19,20-tetranor-prostansäure,<br>IR: 1710, 1740, 3400 $cm^{-1}$;<br>NMR: 1,65; 2,3; 4,25; 7,3; |
| 279 | 11,15-Dihydroxy-9-oxo-15-(4-methoxyphenyl)-13-thia-17,18,19,20-tetranor-prostansäure, |
| 280 | 11,15-Dihydroxy-9-oxo-15-(4-tolyl)-13-thia-17,18,19,20-tetranor-prostansäure,<br>IR: 1510, 1707, 1740, 3400 $cm^{-1}$;<br>NMR: 1,6; 2,3; 4,2; 7,1–7,5; |
| 281 | 11,15-Dihydroxy-9-oxo-15-(3-trifluormethylphenyl)-13-thia-17,18,19,20-tetranor-prostansäure, |
| 282 | 11,15-Dihydroxy-9-oxo-15-(3,4,5-trimethoxyphenyl)-13-thia-17,18,19,20-tetranor-prostansäure,<br>IR: 1130, 1600, 1670, 1710, 1740, 2950, 3450 $cm^{-1}$;<br>NMR: 1,63; 2,3; 3,8; 4,23; 5,4; 6,63; |

Fortsetzung

| Beispiel | Verbindung der Formel III mit D = —CO— und E = $-CH_2CH_2-$ |
|---|---|
| 283 | 11,15-Dihydroxy-9-oxo-15-methyl-16-phenyl-13-thia-17,18,19,20-tetranor-prostansäure, |
| | IR: 1500, 1715, 1745, 3450 $cm^{-1}$; |
| | NMR: 1,2; 2,35; 5,0; 7,3; |
| 284 | 11,15-Dihydroxy-9-oxo-15-methyl-16-(4-fluorphenyl)-13-thia-17,18,19,20-tetranor-prostansäure, |
| 285 | 11,15-Dihydroxy-9-oxo-15-methyl-16-(4-chlorphenyl)-13-thia-17,18,19,20-tetranor-prostansäure, |
| 286 | 11,15-Dihydroxy-9-oxo-15-methyl-16-(4-tolyl)-13-thia-17,18,19,20-tetranor-prostansäure, |
| 287 | 11,15-Dihydroxy-9-oxo-15-methyl-16-(3-chlorphenyl)-13-thia-17,18,19,20-tetranor-prostansäure, |
| | IR: 1400, 1495, 1705, 1740, 2950, 3450 $cm^{-1}$; |
| | NMR: 1,25; 2,35; 4,3; 5,6; 7,0—7,4; |
| 288 | 11,15-Dihydroxy-9-oxo-15-methyl-16-(3-trifluormethyl-phenyl)-13-thia-17,18,19,20-tetranor-prostansäure, |
| 289 | 11,15-Dihydroxy-9-oxo-17-phenyl-13-thia-18,19,20-trinor-prostansäure, |
| 290 | 11,15-Dihydroxy-9-oxo-15-methyl-17-phenyl-13-thia-18,19,20-trinor-prostansäure, |
| | IR: 1710, 1730, 3400 $cm^{-1}$; |
| | NMR: 1,35; 5,3; 7,25; |
| 291 | 11,15-Dihydroxy-9-oxo-15-(2-naphtyl)-13-thia-17,18,19,20-tetranor-prostansäure, |
| | IR: 1600, 1705, 1740, 2900, 3400 $cm^{-1}$; |
| | NMR: 1,76; 2,28; 4,2; 7,42; 7,8; |
| 292 | 11,15-Dihydroxy-9-oxo-16-(4-pyridyl)-13-thia-17,18,19,20-tetranor-prostansäure, |
| 293 | 11,15-Dihydroxy-9-oxo-15-(2-thienyl)-13-thia-17,18,19,20-tetranor-prostansäure. |

Analog Beispiel 256 sind aus Verbindungen der Formel III mit D = —CHOH— und E = —CH=CH— durch Oxidation die entsprechenden Verbindungen mit D = —CO— erhältlich, insbesondere die in den folgenden Beispielen 294 bis 306 genannten Verbindungen:

| Beispiel | Verbindung der Formel III mit D = —CO— und E = —CH=CH— |
|---|---|
| 294 | 11,15-Dihydroxy-9-oxo-16-(3-chlorphenoxy)-13-thia-17,18,19,20-tetranor-5-prostensäure, |
| 295 | 11,15-Dihydroxy-9-oxo-15-methyl-16-phenoxy-13-thia-17,18,19,20-tetranor-5-prostensäure, |
| 296 | 11,15-Dihydroxy-9-oxo-15-methyl-16-(4-fluorphenoxy)-13-thia-17,18,19,20-tetranor-5-prostensäure, |
| 297 | 11,15-Dihydroxy-9-oxo-15-methyl-16-(4-tolyloxy)-13-thia-17,18,19,20-tetranor-5-prostensäure, |
| 298 | 11,15-Dihydroxy-9-oxo-15-methyl-13-thia-5-prostensäure, |
| | IR: 1710, 1740, 2900, 3400 $cm^{-1}$; |
| | NMR: 1,27; 2,32; 4,22; 5,43; |
| 299 | 11,15-Dihydroxy-9-oxo-15-phenyl-13-thia-17,18,19,20-tetranor-5-prostensäure, |
| 300 | 11,15-Dihydroxy-9-oxo-15-(4-fluorphenyl)-13-thia-17,18,19,20-tetranor-5-prostensäure, |
| 301 | 11,15-Dihydroxy-9-oxo-15-(4-tolyl)-13-thia-17,18,19,20-tetranor-5-prostensäure, |
| 302 | 11,15-Dihydroxy-9-oxo-15-methyl-16-phenyl-13-thia-17,18,19,20-tetranor-5-prostensäure, |
| 303 | 11,15-Dihydroxy-9-oxo-15-methyl-16-(4-fluorphenyl)-13-thia-17,18,19,20-tetranor-5-prostensäure, |
| 304 | 11,15-Dihydroxy-9-oxo-15-methyl-16-(3-trifluormethylphenyl)-13-thia-17,18,19,20-tetranor-5-prostensäure, |
| 305 | 11,15-Dihydroxy-9-oxo-17-phenyl-13-thia-18,19,20-trinor-5-prostensäure, |
| 306 | 11,15-Dihydroxy-9-oxo-15-methyl-17-phenyl-13-thia-18,19,20-trinor-5-prostensäure. |

## Beispiel 307

Man versetzt 100 mg 9,11,15-Trihydroxy-16-(3-chlorphenoxy)-13-thia-17,18,19,20-tetranor-5-prostensäure, gelöst in 10 ml Diäthyläther, mit überschüssiger ätherischer Diazomethanlösung, bis keine Stickstoffentwicklung mehr festzustellen ist. Das Lösungsmittel wird abdestilliert, und nach chromatographischer Reinigung (Kieselgel/Benzol: Chloroform = 1:1) des Rückstandes erhält man 9,11,15-Trihydroxy-16-(3-chlorphenoxy)-13-thia-17,18,19,20-tetranor-5-prostensäuremethylester.

Analog Beispiel 307 sind aus den nach den Beispielen 152 bis 306 herstellbaren Verbindungen der Formel III durch Umsetzen mit Diazomethan die entsprechenden Methylester erhältlich, insbesondere die in den folgenden Beispielen 308 bis 346 genannten Methylester:

| Beispiel | Methylester der Formel III |
|---|---|
| 308 | 9,11,15-Trihydroxy-16-(4-fluorphenoxy)-13-thia-17,18,19,20-tetranor-5-prostensäuremethylester, |
| 309 | 9,11,15-Trihydroxy-16-(3-trifluormethylphenoxy)-13-thia-17,18,19,20-tetranor-5-prostensäuremethylester, |
| 310 | 9,11,15-Trihydroxy-15-methyl-16-(4-tolyloxy)-13-thia-17,18,19,20-tetranor-5-prostensäuremethylester, |
| 311 | 9,11,15-Trihydroxy-15-methyl-16-(3-chlorphenoxy)-13-thia-17,18,19,20-tetranor-5-prostensäuremethylester, |
| 312 | 9,11,15-Trihydroxy-15-methyl-13-thia-5-prostensäuremethylester, |
| 313 | 9,11,15-Trihydroxy-16-methyl-13-thia-5-prostensäuremethylester, |
| 314 | 9,11,15-Trihydroxy-15,16-dimethyl-13-thia-17,18,19,20-tetranor-5-prostensäuremethylester, |
| 315 | 9,11,15-Trihydroxy-15-phenyl-13-thia-17,18,19,20-tetranor-5-prostensäuremethylester, |
| 316 | 9,11,15-Trihydroxy-15-(4-fluorphenyl)-13-thia-17,18,19,20-tetranor-5-prostensäuremethylester, |
| 317 | 9,11,15-Trihydroxy-15-(4-methoxyphenyl)-13-thia-17,18,19,20-tetranor-5-prostensäuremethylester, |
| 318 | 9,11,15-Trihydroxy-15-methyl-16-(3-chlorphenyl)-13-thia-17,18,19,20-tetranor-5-prostensäuremethylester, |
| 319 | 9,11,15-Trihydroxy-15-methyl-17-phenyl-13-thia-18,19,20-trinor-5-prostensäuremethylester, |
| 320 | 9,11,15-Trihydroxy-15-methyl-17-(3-chlorphenyl)-13-thia-18,19,20-trinor-5-prostensäuremethylester, |
| 321 | 9,11,15-Trihydroxy-15-methyl-17-(3-trifluormethylphenyl-13-thia-18,19,20-trinor-5-prostensäuremethylester, |
| 322 | 9,11,15-Trihydroxy-16-phenoxy-13-thia-17,18,19,20-tetranor-prostensäuremethylester, |
| 323 | 9,11,15-Trihydroxy-15-methyl-16-(4-fluorphenoxy)-13-thia-17,18,19,20-tetranor-prostansäuremethylester, |
| 324 | 9,11,15-Trihydroxy-15-methyl-13-thia-prostansäuremethylester,<br>IR: 1740, 3490 $cm^{-1}$;<br>NMR: 0,90; 1,22; 2,31; 3,61; 4,18; |
| 325 | 9,11,15-Trihydroxy-13-thia-prostansäuremethylester, |
| 326 | 9,11,15-Trihydroxy-15,16-dimethyl-13-thia-prostansäuremethylester, |
| 327 | 9,11,15-Trihydroxy-15-(4-tolyl)-13-thia-17,18,19,20-tetranor-prostansäuremethylester, |
| 328 | 9,11,15-Trihydroxy-15-methyl-16-(4-fluorphenyl)-13-thia-17,18,19,20-tetranor-prostansäuremethylester, |

Fortsetzung

| Beispiel | Methylester der Formel III |
|---|---|
| 329 | 9,11,15-Dihydroxy-9-oxo-16-(3-trifluormethylphenoxy)-13-thia-17,18,19,20-tetranor-prostansäuremethylester, |
| 330 | 9,11,15-Dihydroxy-9-oxo-15-methyl-16-(4-fluorphenoxy)-13-thia-17,18,19,20-tetranor-prostansäuremethylester, |
| 331 | 11,15-Dihydroxy-9-oxo-15-methyl-13-thia-prostansäuremethylester, |
| | IR: 1740, 3420 $cm^{-1}$; |
| | NMR: 0,86; 1,21; 2,25; 3,61; 4,22; |
| 332 | 11,15-Dihydroxy-9-oxo-13-thia-prostansäuremethylester, |
| 333 | 11,15-Dihydroxy-9-oxo-15,16-dimethyl-13-thia-prostansäuremethylester, |
| 334 | 11,15-Dihydroxy-9-oxo-17-äthyl-13-thia-prostansäuremethylester, |
| 335 | 11,15-Dihydroxy-9-oxo-15-(4-tolyl)-13-thia-16,17,18,19,20-pentanor-prostansäuremethylester, |
| 336 | 11,15-Dihydroxy-9-oxo-15-phenyl-13-thia-17,18,19,20-tetranor-prostansäuremethylester, |
| 337 | 11,15-Dihydroxy-9-oxo-15-(4-fluorphenyl)-13-thia-17,18,19,20-tetranor-prostansäuremethylester, |
| 338 | 11,15-Dihydroxy-9-oxo-15-(4-chlorphenyl)-13-thia-17,18,19,20-tetranor-prostansäuremethylester, |
| 339 | 11,15-Dihydroxy-9-oxo-15-(4-tolyl)-13-thia-17,18,19,20-tetranor-prostansäuremethylester, |
| 340 | 11,15-Dihydroxy-9-oxo-15-(3,4,5-trimethoxyphenyl)-13-thia-17,18,19,20-tetranor-prostansäuremethylester, |
| 341 | 11,15-Dihydroxy-9-oxo-15-methyl-16-phenyl-13-thia-17,18,19,20-tetranor-prostansäuremethylester, |
| 342 | 11,15-Dihydroxy-9-oxo-16-(3-chlorphenyl)-13-thia-17,18,19,20-tetranor-prostansäuremethylester, |
| 343 | 11,15-Dihydroxy-9-oxo-15-(2-naphthyl)-13-thia-17,18,19,20-tetranor-prostansäuremethylester, |
| 344 | 11,15-Dihydroxy-9-oxo-15-(4-pyridyl)-13-thia-17,18,19,20-tetranor-prostansäuremethylester, |
| | IR: 1600, 1740; 3000–3500 $cm^{-1}$; |
| | NMR: 1,63; 2,28; 3,7; 4,29; 7,33; 8,45; |
| 345 | 11,15-Dihydroxy-9-oxo-16-(3-chlorphenoxy)-13-thia-17,18,19,20-tetranor-5-prostensäuremethylester, |
| 346 | 11,15-Dihydroxy-9-oxo-15-methyl-13-thia-5-prostensäuremethylester, |
| | IR: 1710, 1740, 2900, 3400 $cm^{-1}$; |
| | NMR: 1,27; 2,32; 3,7; 4,22; 5,43; |

## Beispiel 347

Eine Lösung von 1 g 9,11,15-Trihydroxy-15-methyl-13-thia-prostansäure in 10 ml Äthanol wird bei 0°C mit HCl gesättigt. Man läßt 12 Stunden bei 20° stehen, filtriert ab und dampft das Filtrat ein. Der Rückstand wird zwischen Dichlormethan und gesättigter Natriumbikarbonat-Lösung verteilt. Man trennt die organische Phase ab, trocknet sie über $MgSO_4$, filtriert, zieht das Lösungsmittel ab und erhält nach chromatographischer Reinigung 9,11,15-Trihydroxy-15-methyl-13-thia-prostansäureäthylester.

Analog Beispiel 347 sind durch Umsetzen mit n-Propanol, Isopropanol, n-Butanol oder Isobutanol erhältlich:

348: 9,11,15-Trihydroxy-15-methyl-13-thia-prostansäure-n-propylester,
349: 9,11,15-Trihydroxy-15-methyl-13-thia-prostansäure-isopropylester,
350: 9,11,15-Trihydroxy-15-methyl-13-thia-prostansäure-n-butylester,
351: 9,11,15-Trihydroxy-15-methyl-13-thia-prostansäure-isobutylester.

## Beispiel 352

Man kühlt ein Gemisch aus 1,24 g 9α,11α,15-Trihydroxy-16-(3-chlorphenoxy)-13-thia-17,18,19,20-tetranor-5-prostensäure, 0,46 ml Triäthylamin und 40 ml Aceton auf —20°, tropft 0,432 ml Isobutylchlorformiat zu, erwärmt nach 5 Minuten auf 25°, tropft 0,8 g p-Benzoylaminophenol, gelöst in 20 ml trockenem Pyridin zu, rührt 2 Stunden bei Raumtemperatur, destilliert das Lösungsmittel ab, nimmt den Rückstand in Äthylacetat auf, wäscht die organische Phase mit Wasser, trocknet über $Na_2SO_4$, destilliert das Lösungsmittel ab und erhält nach chromatographischer Reinigung des Rückstands (Kieselgel/Äthylacetat) 9α,11α,15-Trihydroxy-16-(3-chlorphenoxy)-13-thia-17,18,19,20-tetranor-5-prostensäure-p-benzoylaminophenylester.

Analog Beispiel 352 sind durch Umsetzen des aus 9α,11α,15-Trihydroxy-16-(3-chlorphenoxy)-13-thia-17,18,19,20-tetranor-5-prostensäure und Isobutylformiat hergestellten gemischten Anhydrids mit dem entsprechenden Phenol der Formel VI die in den folgenden Beispielen 353 bis 360 genannten Verbindungen der Formel III erhältlich:

| Beispiel | Verbindung der Formel III |
|---|---|
| 353 | 9α,11α,15-Trihydroxy-16-(3-chlorphenoxy)-13-thia-17,18,19,20-tetranor-5-prostensäure-p-acetylamino-phenylester, |
| 354 | 9α,11α,15-Trihydroxy-16-(3-chlorphenoxy)-13-thia-17,18,19,20-tetranor-5-prostensäure-p-(p-acetylamino)-phenylester, |
| 355 | 9α,11α,15-Trihydroxy-16-(3-chlorphenoxy)-13-thia-17,18,19,20-tetranor-5-prostensäure-p-(p-benzoyl-amino-benzoylamino)-phenylester, |
| 356 | 9α,11α,15-Trihydroxy-16-(3-chlorphenoxy)-13-thia-17,18,19,20-tetranor-5-prostensäure-p-ureido-phenylester, |
| 357 | 9α,11α,15-Trihydroxy-16-(3-chlorphenoxy)-13-thia-17,18,19,20-tetranor-5-prostensäure-p-(3-phenylureido)-phenylester, |
| 358 | 9α,11α,15-Trihydroxy-16-(3-chlorphenoxy)-13-thia-17,18,19,20-tetranor-5-prostensäure-(4-acetylamino-1-naphthyl)-ester, |
| 359 | 9α,11α,15-Trihydroxy-16-(3-chlorphenoxy)-13-thia-17,18,19,20-tetranor-5-prostensäure-(4-benzoylamino-1-naphthyl)-ester, |
| 360 | 9α,11α,15-Trihydroxy-16-(3-chlorphenoxy)-13-thia-17,18,19,20-tetranor-5-prostensäure-(4-ureido-1-naphthyl)-ester. |

## Beispiel 361

Analog Beispiel 352 erhält man aus 11α,15-Dihydroxy-9-oxo-15-methyl-13-thia-prostansäure durch Umsetzen mit Benzoylaminophenol 11α,15-Dihydroxy-9-oxo-15-methyl-13-thia-prostansäurebenzoylaminophenylester, F = 74—76°C (aus Diäthyläther).

Beispiel 362

0,1 g 9,11,15-Trihydroxy-16-(3-chlorphenoxy)-13-thia-17,18,19,20-tetranor-5-prostensäuremethylester werden 90 Stunden in einem Gemisch aus 2 ml einer wäßrigen gesättigten NaCN-Lösung und 6 ml Methanol gerührt. Man sättigt mit NaCl, extrahiert mit Chloroform, wäscht die organische Phase mit Wasser, trocknet über $MgSO_4$, destilliert das Lösungsmittel ab und erhält nach chromatographischer Reinigung des Rückstandes über Kieselgel 9,11,15-Trihydroxy-16-(3-chlorphenoxy)-13-thia-17,18,19,20-tetranor-5-prostensäure.

IR: 1710, 2400, 3650 $cm^{-1}$;
NMR: 1,1–2,6; 2,6–3,1; 4,05; 4,2; 5,3; 5,4; 6,6–7,4.

Beispiel 363

Man tropft zu einer äthanolischen Natriumäthanolat-Lösung, hergestellt aus 0,24 g Natrium und 20 ml trockenem Äthanol, 4,5 g 9,11,15-Trihydroxy-16-(3-chlorphenoxy)-13-thia-17,18,19,20-tetranor-5-prostensäure, gelöst in 20 ml trockenem Diäthyläther, destilliert das Lösungsmittel ab und erhält als Rückstand das Natriumsalz der 9,11,15-Trihydroxy-16-(3-chlorphenoxy)-13-thia-17,18,19,20-tetranor-5-prostensäure.

Beispiel 364

Analog Beispiel 363 erhält man bei Verwendung einer äthanolischen Calciumäthanolat-Lösung, hergestellt aus 0,40 g Calcium und 20 ml trockenem Äthanol, das Calciumsalz der 9,11,15-Trihydroxy-16-(3-chlorphenoxy)-13-thia-17,18,19,20-tetranor-5-prostensäure.

Die nachstehenden Beispiele betreffen Mischungen von Verbindungen der Formel III mit in der Pharmazie üblichen Träger- oder Hilfsstoffen, welche vor allem als Arzneimittel verwendet werden können:

Beispiel A

Tabletten

Ein Gemisch, bestehend aus 30 g 11α,15-Dihydroxy-15-methyl-9-oxo-13-thiaprostansäure-p-benzoylaminophenylester, 50 g Lactose, 16 g Maisstärke, 2 g Cellulosepulver und 2 g Magnesiumstearat, wird in üblicher Weise zu Tabletten gepreßt, derart, daß jede Tablette 10 mg des Wirkstoffes enthält.

Beispiel B

Dragees

Analog Beispiel A werden Tabletten gepreßt, die anschließend in üblicher Weise mit einem Überzug, bestehend aus Zucker, Maisstärke, Talk und Tragant, überzogen werden.

Beispiel C

Injektionslösung

10 g 9α,11α,15-Trihydroxy-16-(3-chlorphenoxy)-13-thia-17,18,19,20-tetranor-5-prostensäure werden in einem Gemisch aus 4 l destilliertem Wasser, 0,5 l Äthanol und 0,5 l Propylenglycol gelöst, die Lösung wird steril filtriert. Die erhaltene Injektionslösung wird je nach Bedarf in Ampullen, enthaltend 2,5 ml, 5 ml oder 10 ml der Injektionslösung, gefüllt.

Analog sind Tabletten, Dragees und Injektionslösungen erhältlich, die einen oder mehrere der übrigen Wirkstoffe der Formel III enthalten.

**Patentansprüche**

1. Verbindungen der allgemeinen Formel I

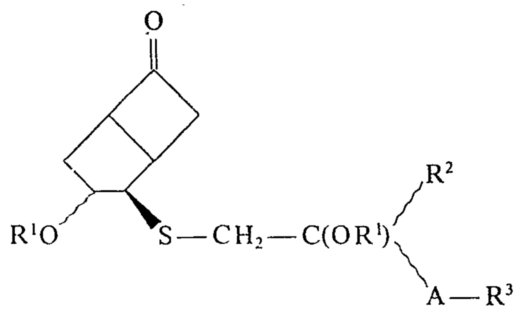

(I)

worin

A eine C–C-Einfachbindung, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ oder $-CH_2O-$,
$R^1$ Wasserstoff oder eine solvolytisch oder hydrogenolytisch abspaltbare Schutzgruppe,
$R^2$ H oder Alkyl mit 1 bis 3 C-Atomen,
$R^3$ Alkyl mit 3 bis 5 C-Atomen; Phenyl oder ein- bis dreifach durch F, Cl, OH, $OCH_3$, $OC_2H_5$, $CF_3$ oder Alkyl mit 1 bis 3 C-Atomen substituiertes Phenyl; oder, wenn A nicht $-CH_2O-$ ist, zusätzlich auch Pyridyl; Thienyl; Naphthyl oder Alkoxy mit 1 bis 4 C-Atomen bedeuten,

◄ eine $\beta$-ständige Bindung anzeigt und eine Wellenlinie (~~~) bedeutet, daß diese Bindungen $\alpha$- oder $\beta$-ständig sein können.

2. 3$\alpha$-Hydroxy-2$\beta$-(3-(3-chlorphenoxy)-2-hydroxy-propylthio)-1$\beta$H,5$\beta$H-bicyclo(3.2.0)heptan-6-on.
3. 3$\alpha$-Hydroxy-2$\beta$-(2-hydroxy-2-methyl-heptylthio)-1$\beta$H,5$\beta$H-bicyclo(3.2.0)heptan-6-on.
4. Verfahren zur Herstellung einer Verbindung der allgemeinen Formel I

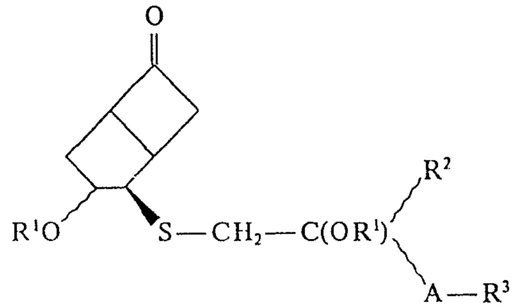

(I)

worin

A eine C–C-Einfachbindung, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ oder $-CH_2O-$,
$R^1$ Wasserstoff oder eine solvolytisch oder hydrogenolytisch abspaltbare Schutzgruppe,
$R^2$ H oder Alkyl mit 1 bis 3 C-Atomen,
$R^3$ Alkyl mit 3 bis 5 C-Atomen; Phenyl oder ein- bis dreifach durch F, Cl, OH, $OCH_3$, $OC_2H_5$, $CF_3$ oder Alkyl mit 1 bis 3 C-Atomen substituiertes Phenyl; oder, wenn A nicht $-CH_2O-$ ist, zusätzlich auch Pyridyl; Thienyl; Naphthyl oder Alkoxy mit 1 bis 4 C-Atomen bedeuten,

◄ eine $\beta$-ständige Bindung anzeigt und eine Wellenlinie (~~~) bedeutet, daß die Bindungen $\alpha$- oder $\beta$-ständig sein können,

dadurch gekennzeichnt, daß man 2-Brom-3-hydroxy-bicyclo(3.2.0)heptan-6-on mit einem Thiol oder Thiolat der allgemeinen Formel II

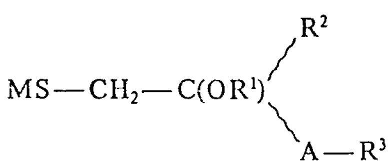

(II)

worin

M H, ein Äquivalent eines Metallatoms oder Ammonium bedeutet und
A und $R^1$ bis $R^3$ die oben angegebenen Bedeutungen haben,

umsetzt
und daß man gegebenenfalls eine oder mehrere vorhandene Hydroxygruppe(n) durch eine solvolytisch oder hydrogenolytisch abspaltbare Schutzgruppe blockiert.

5. Verwendung einer Verbindung der allgemeinen Formel I zur Herstellung von 13-Thiaprostaglandin-Derivaten der allgemeinen Formel III

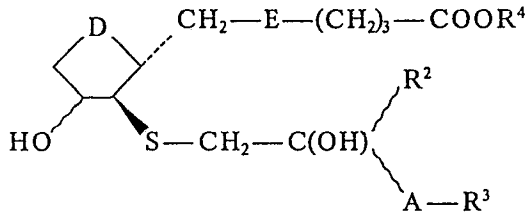

(III)

worin

D —CO— oder —CHOH—,
E $-CH_2-CH_2-$ oder —CH=CH—,
$R^4$ H, Alkyl mit 1 bis 4 C-Atomen oder $-Q-NH-COR^5$,
Q 1,4-Phenylen oder 1,4-Naphthylen und
$R^5$ $NH_2$, $CH_3$, Phenyl, p-Acetylaminophenyl, p-Benzoylaminophenyl oder Phenylamino bedeuten,

A, $R^2$ und $R^3$ die oben angegebenen Bedeutungen haben,
... eine $\alpha$-ständige Bindung und ◄ eine $\beta$-ständige Bindung anzeigt und eine Wellenlinie (~~~) bedeutet, daß diese Bindungen $\alpha$- oder $\beta$-ständig sein können,

sowie ihrer physiologisch unbedenklichen Salze, wenn $R^4$ Wasserstoff bedeutet,
dadurch gekennzeichnet, daß man eine Verbindung der allgemeinen Formel I ultraviolett bestrahlt und daß man das erhaltene Lactol der allgemeinen Formel IV

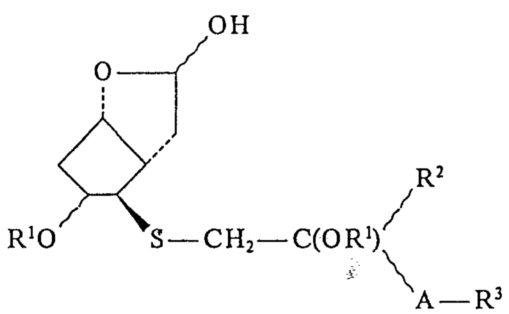

(IV)

worin

A und $R^1$ bis $R^3$ die oben angegebenen Bedeutungen haben,
... eine $\alpha$-ständige Bindung und ◄ eine $\beta$-ständige Bindung anzeigt und eine Wellenlinie (~~~) bedeutet, daß diese Bindungen $\alpha$- oder $\beta$-ständig sein können,

mit einer Verbindung der allgemeinen Formel V

$$[(R^6)_3P^{(+)}-(CH_2)_4-COOR^4]X^{(-)} \qquad V$$

worin

$R^6$ Alkyl mit 1—4 C-Atomen oder Phenyl,
X Cl, Br oder J bedeutet und
$R^4$ die oben angegebene Bedeutung hat,

umsetzt,
und daß man gegebenenfalls eine Verbindung der Formel III mit E = —CH=CH— durch Hydrierung in eine Verbindung der Formel III mit E = $-CH_2-CH_2-$ überführt und/oder vorhandene Schutzgruppen in $C_{11}$-

und $C_{15}$-Stellung solvolytisch oder hydrogenolytisch abspaltet und/oder daß man zur Herstellung der 13-Thiaprostaglandin-E-Analogen der allgemeinen Formel III auf einer beliebigen Stufe die Hydroxygruppen in $C_{11}$- und $C_{15}$-Stellung selektiv durch solvolytisch oder hydrogenolytisch abspaltbare Schutzgruppen blockiert, die freie Hydroxygruppe in $C_9$-Stellung zur Ketogruppe oxidiert und die Schutzgruppen in $C_{11}$- und $C_{15}$-Stellung solvolytisch oder hydrogenolytisch abspaltet,
und/oder daß man gegebenenfalls eine Verbindung der Formel III mit $R^4 = H$ durch Umsetzen mit einem entsprechenden veresternden Mittel in eine andere Verbindung der Formel III mit $R^4$ = Alkyl mit 1 bis 4 C-Atomen umwandelt,
und/oder daß man gegebenenfalls eine Verbindung der Formel III mit $R^4 = H$ durch Umsetzen mit einer Verbindung der Formel VI

$$HO{-}Q{-}NH{-}COR^5 \qquad VI$$

in eine andere Verbindung der Formel III mit $R^4 = {-}Q{-}NH{-}COR^5$ umwandelt,
und/oder daß man gegebenenfalls eine Verbindung der Formel III mit $R^4$ = Alkyl mit 1–4 C-Atomen oder $-Q-NH-COR^5$ durch Umsetzen mit einem solvolysierenden Mittel in eine andere Verbindung der Formel III mit $R^4 = H$ umwandelt,
und/oder daß man gegebenenfalls eine Verbindung der Formel III in ihre Racemate und/oder Enantiomeren aufspaltet,
und/oder daß man gegebenenfalls eine Verbindung der Formel III mit $R^4 = H$ durch Behandeln mit einer Base in eines ihrer physiologisch unbedenklichen Salze umwandelt oder aus einem ihrer Salze durch Behandeln mit einer Säure in Freiheit setzt.

6. Verwendung nach Anspruch 5, dadurch gekennzeichnet, daß man 3$\alpha$-Hydroxy-2$\beta$-(3-(3-chlorphenoxy)-2-hydroxypropylthio)-1$\beta$H,5$\beta$H-bicyclo(3.2.0)heptan-6-on ultraviolett bestrahlt, das erhaltene 2-Oxa-3-hydroxy-6$\beta$-(3-(3-chlorphenoxy)-2-hydroxy-propylthio)-7$\alpha$-hydroxy-1$\beta$H,5$\beta$H-bicyclo-(3.2.0)octan mit Carboxybutyltriphenylphosphonium umsetzt und so 9$\alpha$,11$\alpha$,15-Trihydroxy-16-(3-chlorphenoxy)-13-thia-17,18,19,20-tetranor-(Z)-5-prostensäure erhält.

7. Verfahren zur Herstellung von 13-Thiaprostaglandin-Derivaten der allgemeinen Formel III

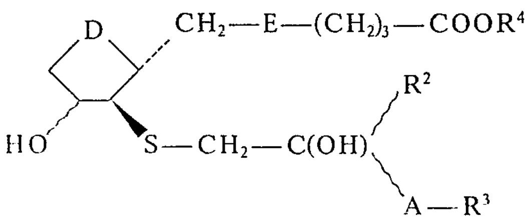

(III)

worin

D $-CO-$ oder $-CHOH-$,
E $-CH_2-CH_2-$ oder $-CH=CH-$,
$R^4$ H, Alkyl mit 1 bis 4 C-Atomen oder $-Q-NH-COR^5$,
Q 1,4-Phenylen oder 1,4-Naphthylen und
$R^5$ $NH_2$, $CH_3$, Phenyl, p-Acetylaminophenyl, p-Benzoylaminophenyl oder Phenylamino bedeuten,

A, $R^2$ und $R^3$ die oben angegebenen Bedeutungen haben,
... eine $\alpha$-ständige Bindung und ◀ eine $\beta$-ständige Bindung anzeigt und eine Wellenlinie (~~) bedeutet, daß diese Bindungen $\alpha$- oder $\beta$-ständig sein können,

sowie ihrer physiologisch unbedenklichen Salze, wenn $R^4$ Wasserstoff bedeutet,
dadurch gekennzeichnet, daß man eine Verbindung der allgemeinen Formel I ultraviolett bestrahlt und daß man das erhaltene Lactol der allgemeinen Formel IV

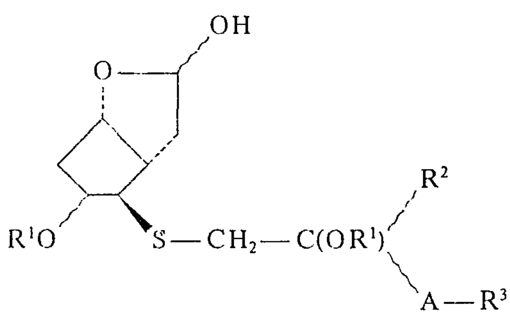

(IV)

worin

A und $R^1$ bis $R^3$ die oben angegebenen Bedeutungen haben, ... eine $\alpha$-ständige Bindung und ◀ eine $\beta$-ständige Bindung anzeigt und eine Wellenlinie (〰) bedeutet, daß diese Bindungen $\alpha$- oder $\beta$-ständig sein können,

mit einer Verbindung der allgemeinen Formel V

$$[(R^6)_3P^{(+)}-(CH_2)_4-COOR^4]X^{(-)} \qquad V$$

worin

$R^6$ Alkyl mit 1—4 C-Atomen oder Phenyl,
X Cl, Br oder J bedeutet und
$R^4$ die oben angegebene Bedeutung hat,

umsetzt,
und daß man gegebenenfalls eine Verbindung der Formel III mit E = —CH=CH— durch Hydrierung in eine Verbindung der Formel III mit E = $—CH_2—CH_2—$ überführt und/oder vorhandene Schutzgruppen in $C_{11}$- und $C_{15}$-Stellung solvolytisch oder hydrogenolytisch abspaltet und/oder daß man zur Herstellung der 13-Thiaprostaglandin-E-Analogen der allgemeinen Formel III auf einer beliebigen Stufe die Hydroxygruppen in $C_{11}$- und $C_{15}$-Stellung selektiv durch solvolytisch oder hydrogenolytisch abspaltbare Schutzgruppen blockiert, die freie Hydroxygruppe in $C_9$-Stellung zur Ketogruppe oxidiert und die Schutzgruppen in $C_{11}$- und $C_{15}$-Stellung solvolytisch oder hydrogenolytisch abspaltet,
und/oder daß man gegebenenfalls eine Verbindung der Formel III mit $R^4$ = H durch Umsetzen mit einem entsprechenden veresternden Mittel in eine andere Verbindung der Formel III mit $R^4$ = Alkyl mit 1 bis 4 C-Atomen umwandelt,
und/oder daß man gegebenenfalls eine Verbindung der Formel III mit $R^4$ = H durch Umsetzen mit einer Verbindung der Formel VI

$$HO—Q—NH—COR^5 \qquad VI$$

in eine andere Verbindung der Formel III mit $R^4$ = —Q—NH—$COR^5$ umwandelt,
und/oder daß man gegebenenfalls eine Verbindung der Formel III mit $R^4$ = Alkyl mit 1—4 C-Atomen oder —Q—NH—$COR^5$ durch Umsetzen mit einem solvolysierenden Mittel in eine andere Verbindung der Formel III mit $R^4$ = H umwandelt,
und/oder daß man gegebenenfalls eine Verbindung der Formel III in ihre Racemate und/oder Enantiomeren aufspaltet,
und/oder daß man gegebenenfalls eine Verbindung der Formel III mit $R^4$ = H durch Behandeln mit einer Base in eines ihrer physiologisch unbedenklichen Salze umwandelt oder aus einem ihrer Salze durch Behandeln mit einer Säure in Freiheit setzt.

8. Verfahren nach Anspruch 7, dadurch gekennzeichnet, daß man 3$\alpha$-Hydroxy-2$\beta$-(3-(3-chlorohenoxy)-2-hydroxypropylthio)-1$\beta$H,5$\beta$H-bicyclo(3.2.0)heptan-6-on ultraviolett bestrahlt, das erhaltene 2-Oxa-3-hydroxy-6$\beta$-(3-(3-chlorphenoxy)-2-hydroxy-propylthio)-7$\alpha$-hydroxy-1$\beta$H,5$\beta$H-bicyclo(3.2.0)octan mit Carboxybutyltriphenylphosphoniumbromid umsetzt und so 9$\alpha$,11$\alpha$,15-Trihydroxy-16-(3-chlorphenoxy)-13-thia-17,18,19,20-tetranor-(Z)-5-prostensäure erhält.

## Claims

1. Compounds of the general formula I

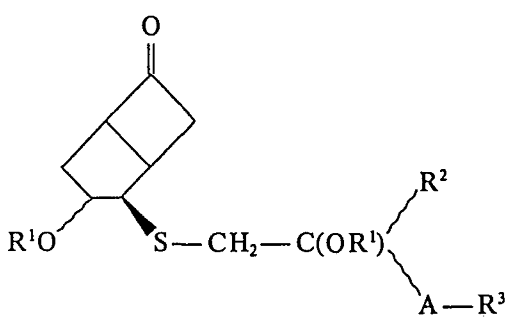

(I)

wherein

A is a C—C single bond, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ or $-CH_2O-$,
$R^1$ is hydrogen or a protective group which can be cleaved by solvolysis or hydrogenolysis,
$R^2$ is H or alkyl having 1 to 3 C atoms and
$R^3$ is alkyl having 3 to 5 C atoms, phenyl or phenyl which is monosubstituted by F, Cl, OH, $OCH_3$, $OC_2H_5$, $CF_3$ or alkyl having 1 to 3 C atoms, or, if A is not $-CH_2O-$, is in addition also pyridiyl, thienyl, naphthyl or alkoxy having 1 ti 4 C atoms,

indications a bond in the $\beta$-position and a wavy line (~) means that these bonds can be in the $\alpha$- or $\beta$-position.

2. $3\alpha$-Hydroxy-$2\beta$-(3-(3-chlorphenoxy)-2-hydroxypropylthio)-$1\beta$H,$5\beta$H-bicyclo(3.2.0)heptan-6-one.

3. $3\alpha$-Hydroxy-$2\beta$-(2-hydroxy-2-methyl-heptylthio)-bicyclo[3.2.0]heptan-6-one.

4. Process for the preparation of a compound of the general formula I

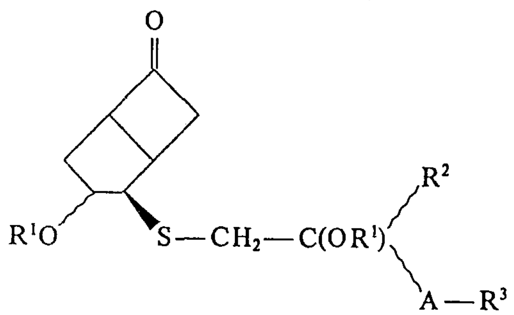

(I)

wherein

A is a C—C single bond, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ or $-CH_2O-$,
$R^1$ is hydrogen or a protective group which can be cleaved by solvolysis or hydrogenolysis,
$R^2$ is H or alkyl having 1 to 3 C atoms and
$R^3$ is alkyl having 3 to 5 C atoms, phenyl or phenyl which is monosubstituted to trisubstituted by F, Cl, OH, $OCH_3$, $OC_2H_5$, $CF_3$ or alkyl having 1 to 3 C atoms, or, if A is not $-CH_2O-$, is in addition also pyridyl, thienyl, naphthyl or alkoxy having 1 to 4 C atoms,

indicates a bond in the $\beta$-position and a wavy line (~) means that these bonds can be in the $\alpha$- or $\beta$-position,

characterised in that 2-bromo-3-hydroxy-bicyclo[3.2.0]heptan-6-one is reacted with a thiol or thiolate of the general formula II

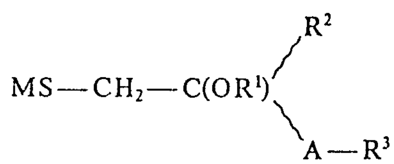

(II)

wherein

M is H, one equivalent of a metal atom or ammonium and
A and $R^1$ to $R^3$ have the meanings indicated above,

and that, if appropriate, one or more available hydroxy groups are blocked by a protective group which can be cleaved by solvolysis or hydrogenolysis.

5. Use of a compound of the general formula I for the preparation of 13-thiaprostaglandin derivates of the general formula III

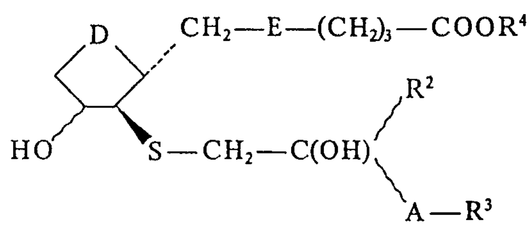

(III)

D is $-CO-$ or $-CHOH-$,
E is $-CH_2-CH_2-$ or $-CH=CH-$,
$R^4$ is H, alkyl having 1 to 4 C atoms or $-Q-NH-COR^5$,
Q is 1,4-phenylene or 1,4-naphthylene and
$R^5$ is $NH_2$, $CH_3$, phenyl, p-acetylaminophenyl, p-benzoylaminophenyl or phenylamino,

A, $R^2$ and $R^3$ have the meanings indicated above,
... indicates a bond in the $\alpha$-position and ◄ indicates a bond in the $\beta$-position and a wavy line (~~~) means that these bonds can be in the $\alpha$- or $\beta$-position, and also of their physiologically acceptable salts if $R^4$ is hydrogen,

characterised in that a compound of the general formula I is irrediated with ultraviolet and in that the resulting lactol of the general formula IV

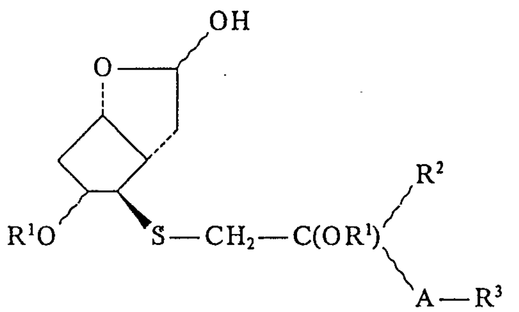

(IV)

wherein

A and $R^1$ to $R^3$ have the meanings indicated above,
... indicates a bond in the $\alpha$-position and ◄ indicates a bond in the $\beta$-position and a wavy line (~~~) means that these bonds can be in the $\alpha$- or $\beta$-position,

is reacted with a compound of the general formula V

$$[(R^6)_3P^{(+)}-(CH_2)_4-COOR^4]X^{(-)} \quad V$$

wherein

$R^6$ is alkyl having 1–4 C atoms or phenyl,
X is Cl, Br or I and
$R^4$ has the meaning indicated above,

and that, if appropriate, a compound of the formula III in which $E = -CH=CH-$ is converted by hydrogenation into a compound of the formula III in which $E = -CH_2-CH_2-$, and/or available protective groups in the $C_{11}$- and $C_{15}$-positions are cleaved by solvolysis or hydrogenolysis, and/or in that, in order to prepare the 13-thiaprostaglandin E-analogues of the general formula III, the hydroxyl groups in the $C_{11}$- and $C_{15}$-positions are selectively blocked, at any desired stage, by protective groups which can be cleaved by solvolysis or hydrogenolysis, the free hydroxyl group in the $C_9$-position is oxidised to a keto group and the protective groups in the $C_{11}$- and $C_{15}$-positions are cleaved by solvolysis or hydrogenolysis, and/or in that, if appropriate, a compound of the formula III in which $R^4 = H$ is converted, by reaction with a corresponding esterifying agent, into another compound of the formula III in which $R^4$ = alkyl having 1 to 4 C atoms, and/or in that, if appropriate, a compound of the formula III in which $R^4 = H$ is converted, by reaction with a compound of the formula VI

$$HO-Q-NH-COR^5 \quad VI$$

into another compound of the formula III in which $R^4 = -Q-NH-COR^5$, and/or in that, if appropriate, a compound of the formula III in which $R^4$ = alkyl having 1–4 C atoms or $-Q-NH-COR^5$ is converted, by reaction with a solvolysing agent, into another compound of the formula III in which $R^4 = H$, and/or in that, if appropriate, a compound of the formula III is split into its racemates and/or enentiomers, and/or in that, if appropriate, a compound of the formula III in which $R^4 = H$ is converted, by treatment with a base, into one of its physiologically acceptable salts or liberated from one of its salts by treatment with an acid.

6. Use according to Claim 5, charaterised in that 3$\alpha$-hydroxy-2$\beta$-(3-(3-chlorphenoxy)-2-hydroxypropylthio)-1$\beta$H,5$\beta$H-bicyclo(3.2.0)heptan-6-one is irradiated with ultraviolett, the resulting 2-oxa-3-hydroxy-6$\beta$-(3-(3-chlorophenoxy)-2-hydroxy-propylthio)-7$\alpha$-hydroxy-1$\beta$H,5$\beta$H-bicyclo(3.2.0)octane is

reacted with carboxybutyltriphenylphosphonium bromide, and 9$\alpha$,11$\alpha$,15-trihydroxy-16-(3-chlorophenoxy)-13-thia-17,18,19,20-tetranor-(Z)-5-prostenoic acid is thus obtained.

7. Process for the preparation of 13-thiaprostaglandin derivates of the general formula III

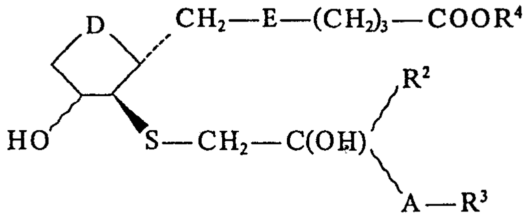

(III)

wherein

D is $-CO-$ or $-CHOH-$,
E is $-CH_2-CH_2-$ or $-CH=CH-$,
$R^4$ is H, alkyl having 1 to 4 C atoms or $-Q-NH-COR^5$,
Q is 1,4-phenylene or 1,4-naphthylene and
$R^5$ is $NH_2$, $CH_3$, phenyl, p-acetylaminophenyl, p-benzoylaminophenyl or phenylamino,

A, $R^2$ and $R^3$ have the meanings indicated above,
... indicates a bond in the $\alpha$-position and ◀ indicates a bond in the $\beta$-position and a wavy line (〰) means that these bonds can be in the $\alpha$- or $\beta$-position, and also of their physiologically acceptable salts if $R^4$ is hydrogen, characterised in that a compound of the general formula I is irrediated with ultraviolet and in that the resulting lactol of the general formula IV

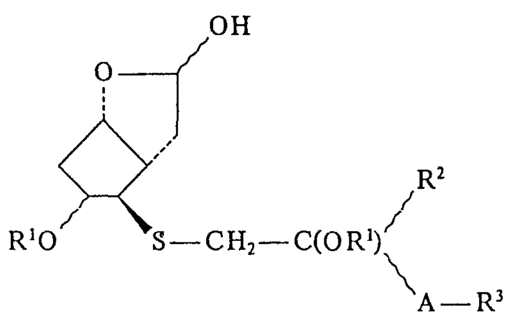

(IV)

wherein

A and $R^1$ to $R^3$ have the meanings indicated above,
... indicates a bond in the $\alpha$-position and ◀ indicates a bond in the $\beta$-position and a wavy line (〰) means that these bonds can be in the $\alpha$- or $\beta$-position,

is reacted with a compound of the general formula V

$$[(R^6)_3P^{(+)}-CCH_2)_4-COOR^4]X^{(-)} \qquad V$$

wherein

$R^6$ is alkyl having 1–4 C atoms or phenyl,
X is Cl, Br or I and
$R^4$ has the meaning indicated above,

and that, if appropriate, a compound of the formula III in which E = $-CH=CH-$ is converted by hydrogenation into a compound of the formula III in which E = $-CH_2-CH_2-$, and/or available protective groups in the $C_{11}$- and $C_{15}$-positions are cleaved by solvolysis or hydrogenolysis, and/or in that, in order to prepare the 13-thiaprostaglandin E-analogues of the general formula III, the hydroxyl groups in the $C_{11}$- and $C_{15}$-positions are selectively blocked, at any desired stage, by protective groups which can be cleaved by solvolysis or hydrogenolysis, the free hydroxyl group in the $C_9$-position is oxidised to a keto group and the protective groups in the $C_{11}$- and $C_{15}$-positions are cleaved by solvolysis or hydrogenolysis, and/or in that, if appropriate, a compound of the formula III in which $R^4$ = H is converted, by reaction with a corresponding esterifying agent, into another compound of the formula III in which $R^4$ = alkyl having 1 to 4 C atoms, and/or in that, if appropriate, a compound of the formula III in which $R^4$ = H is converted, by reaction with a compound of the formula VI

$$HO-Q-NH-COR^5 \qquad VI$$

into another compound of the formula III in which $R^4 = -Q-NH-COR^5$, and/or in that, if appropriate, a compound of the formula III in which $R^4$ = alkyl having 1–4 C atoms or $-Q-NH-COR^5$ is converted, by reaction with a solvolysing agent, into another compound of the formula III in which $R^4 = H$, and/or in that, if appropriate, a compound of the formula III is split into its racemates and/or enantiomers, and/or in that, if appropriate, a compound of the formula III in which $R^4 = H$ is converted, by treatment with a base, into one of its physiologically acceptable salts or liberated from one of its salts by treatment with an acid.

6. Process according to Claim 7, charaterised in that 3α-Hydroxy-2β-(3-(3-chlorphenoxy)-2-hydroxypropylthio)-1βH,5βH-bicyclo(3.2.0)heptan-6-one is irradiated with ultraviolett, the resulting 2-oxa-3-hydroxy-6β-(3-(3-chlorophenoxy)-2-hydroxy-propylthio)-7α-hydroxy-1βH,5βH-bicyclo-(3.2.0)octane is reacted with carboxybutyltriphenylphosphonium bromide, and 9α,11α,15-trihydroxy-16-(3-chlorophenoxy)-13-thia-17,18,19,20-tetranor-(Z)-5-protenoic acid is thus obtained.

## Revendications

1. Composés de formule générale I:

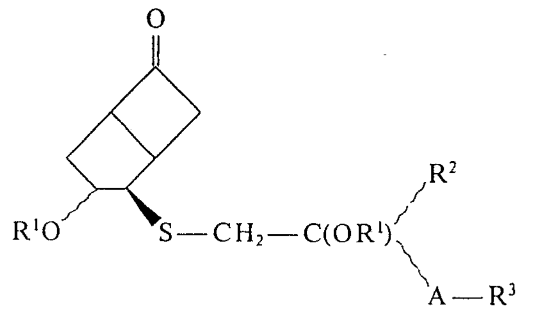

(I)

dans laquelle

A représente une liaison simple C–C, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ ou $-CH_2O-$,
$R^1$ de l'hydrogène ou un groupe protecteur clivable par solvolyse ou hydrogénolyse,
$R^2$ H ou alcoyle ayant l à 3 atomes de carbone,
$R^3$ alcoyle ayant 3 à 5 atomes de carbone; phényle ou phényle substitué une à trois fois par F, Cl, OH, $OCH_3$, $OC_2H_5$, $CF_3$ ou alcoyle ayant l à 3 atomes de carbone, ou bien, lorsque A n'est pas $-CH_2O-$, en plus aussi pyridyle, thiényle, naphtyle ou alcoxy ayant l à 4 atomes de carbone,

◄ indiquant une liaison en position β et une ligne ondulée (∼) signifiant que ces liaisons peuvent être en positon α ou β.

2. 3α-hydroxy-2β-(3-(3-chlorophénoxy)-2-hydroxy-propylthio)-1βH,5βH-bicyclo(3.2.0)heptane-6-one.

3. 3α-hydroxy-2β-(2-hydroxy-2-méthylhepthylthio)-1βH,5βH-bicyclo(3.2.0)heptane-6-one.

4. Procédé de préparation d'un composé de formule générale I:

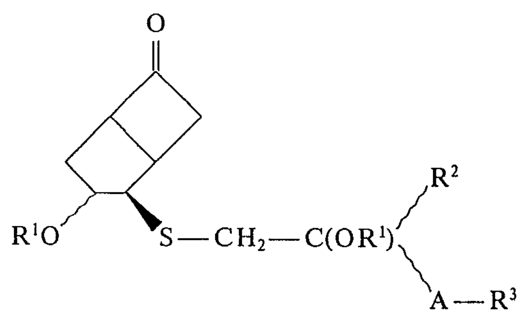

(I)

dans laquelle

A signifie une liaison simple C–C, $-CH_2-$, $-CH(CH_3)-$, $-C(CH_3)_2-$, $-CH_2-CH_2-$, $-CH(CH_3)CH_2-$, $-C(CH_3)_2CH_2-$, $-CH_2-CH_2-CH_2-$ ou $-CH_2O-$,
$R^1$ de l'hydrogène ou un groupe protecteur clivable par solvolyse ou hydrogénolyse,
$R^2$ H ou alcoyle ayant l à 3 atomes de carbone,
$R^3$ alcoyle ayant 3 à 5 atomes de carbone, phényle ou phényle substitué une à trois fois par F, Cl, OH,

$OCH_3$, $OC_2H_5$, $CF_3$ ou alcoyle ayant l à 3 atomes de carbone; ou bien, lorsque A n'est pas $-CH_2O-$, en plus aussi pyridyle; thiényle; naphtyle ou alcoxy ayant l à 4 atomes de carbone,

◄ indiquant une liaison en position $\beta$ et une ligne ondulée (~~~) signifiant que les liaisons peuvent être en position $\alpha$ ou $\beta$.

caractérisé en e qu'on fait réagir de la 2-bromo-3-hydroxy-bicyclo(3.2.0)heptane-6-one avec un thiol ou thiolate de formule générale II:

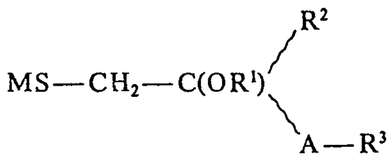

(II)

dans laquelle

M signifie H, un équivalent d'un atome de métal ou d'ammonium et
A et $R^1$ à $R^3$ ont les significations indiquées plus haut

et en ce qu'éventuellement on bloque un ou plusieurs groupe(s) hydroxy présent(s) par un groupe protecteur clivable par solvolyse ou hydrogénolyse.

5. Utilisation d'un composé de formule générale I pour la fabrication de dérivés de 13-thiaprostaglandine de formule générale III

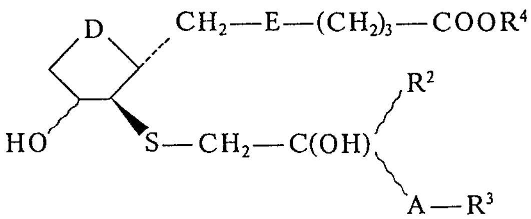

(III)

dans laquelle

D signifie –CO– ou –CHOH–,
E $-CH_2-CH_2-$ ou $-CH=CH-$,
$R^4$ H, alcoyle ayant l à 4 atomes de carbone ou $-Q-NH-COR^5$,
Q 1,4-phénylène ou 1,4-naphtylène et
$R^5$ $NH_2$, $CH_3$, phényle, p-acétylaminophényle, p-benzoylaminophényle ou phénylamino,

A, $R^2$ et $R^3$ ayant les significations indiquées plus haut,
... indiquant une liaison en position $\alpha$ et ◄ une liaison en position $\beta$ tandisqu'une ligne ondulée (~~~) signifie que ces liaisons peuvent être en position $\alpha$ ou $\beta$,

de même que leurs sels physiologiquement inoffensifs, lorsque $R^4$ signifie de l'hydrogène, caractérisé en ce qu'on irradie avec de la lumière ultraviolette un composé de formule générale I et en ce qu'on fait réagir le lactol obtenu de formule générale IV:

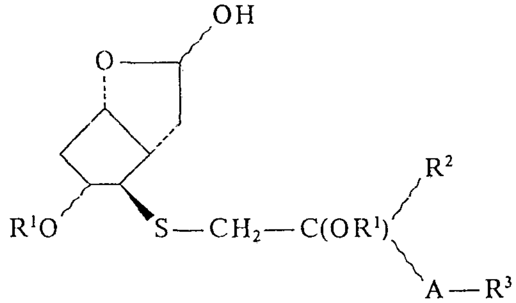

(IV)

dans laquelle

A et $R^1$ à $R^3$ ont les significations indiquées plus haut,
... indiquant une liaison en position $\alpha$ et ◄ une liaison en position $\beta$, tandis qu'une ligne ondulée (~~~) indique que ces liaisons peuvent être en position $\alpha$ ou $\beta$,

avec un composé de formule générale V:

$$[(R^6)_3P^{(+)}-(CH_2)_4-COOR^4]X^{(-)} \qquad V$$

dans laquelle

$R^6$ signifie un alcoyle ayant I à 4 atomes de carbone ou phényle,
X Cl, Br ou I et
$R^4$ a la signification indiquée plus haut,

et en ce qu'éventuellement on convertit un composé de formule III avec $E = -CH=CH-$ par hydrogénation en un composé de formule III avec $E = -CH_2-CH_2-$ et/ou on clive les groupes protecteurs éventuellement en position $C_{11}$ et $C_{15}$ par solvolyse ou hydrogénolyse, et/ou en ce que pour la préparation des analogues de 13-thia-prostaglandine-E de formule générale III, on bloque en un stade quelconque les groupes hydroxy en position $C_{11}$ et $C_{15}$ sélectivement par des groupes protecteurs clivables par solvolyse ou hydrogénolyse, on oxyde le groupe hydroxy libre en position $C_9$ en groupe cétonique et l'on clive les groupes protecteurs en position $C_{11}$ et $C_{15}$ par solvolyse ou hydrogénolyse, et/ou en ce qu'éventuellement on convertit un composé de formule III avec $R^4 = H$, par réaction avec un agent d'estérification correspondant, en un autre composé de formule III avec $R^4 =$ alcoyle ayant I à 4 atomes de carbone, et/ou en ce qu'éventuellement on convertit un composé de formule III avec $R^4 = H$, par réaction avec un compose de formule VI:

$$HO-Q-NH-COR^5 \qquad VI$$

en un autre composé de formule III avec $R^4 = -Q-NH-COR^5$,
et/ou en e qu'éventuellement on convertit un composé de formule III avec $R^4 =$ alcoyle ayant I à 4 atomes de carbone ou $-Q-NH-COR^5$, par réaction avec un agent de solvolyse, en un autre composé de formule III avec $R^4 = H$,
et/ou en ce qu'éventuellement on clive un composé de formule III en ses racémates et/ou énantiomères, et/ou en ce qu'éventuellement on convertit un composé de formule III avec $R^4 = H$, par traitement avec une base, en un de ses sels physiologiquement inoffensifs ou en ce qu'à partir d'un de ses sels on le met en liberté par traitement avec un acide.

6. Utilisation selon la revendication 5, caractérisée en e qu'on irradie avec de la lumière ultraviolette de la 3α-hydroxy-2β-(3-(3-chlorophénoxy)-2-hydroxypropylthio)-1βH,5βH-bicyclo(3.2.0)heptane-6-one, on fait réagir le 2-oxa-3-hydroxy-6β-(3-(3-chlorophénoxy)-2-hydroxy-propylthio)-7α-hydroxy-1βH,5βH-bicyclo(3.3.0)octane obtenu avec du bromure de carboxybutyltriphényl-phosphonium, en obtenant ainsi l'acide 9α,11α,15-trihydroxy-16-(3-chlorophénoxy)-13-thia-17,18,19,20-tétranor-(Z)-5-prostènique.

7. Procédé de préparation de dérivés de 13-thiaprostaglandine de formule générale III:

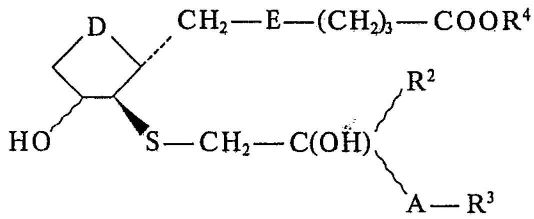

(III)

dans laquelle

D signifie –CO– ou –CHOH–,
E $-CH_2-CH_2-$ ou $-CH=CH-$,
$R^4$ H, alcoyle ayant I à 4 atomes de carbone ou $-Q-NH-COR^5$,
Q 1,4-phénylène ou 1,4-naphtylène et
$R^5$ $NH_2$, $CH_3$, phényle, p-acétylaminophényle, p-benzoylaminophényle ou phénylamino,

A, $R^2$ et $R^3$ ont les significations indiquées plus haut,
. . . indique une liaison en position α et ◀ une liaison en position β, alors qu'une ligne ondulée (~~) signifie que ces liaisons peuvent être en position α ou β,

ainsi que leurs sels physiologiquement inoffensifs, lorsque $R^4$ signifie de l'hydrogène, caractérisé en e qu'on irradie avec de la lumière ultraviolette un composé de formule générale I et en ce qu'on fait réagir le lactol obtenu de formule générale IV:

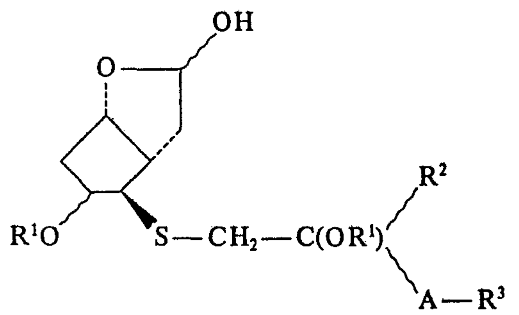

(IV)

dans laquelle

A et $R^1$ à $R^3$ ont les significations indiquées plus haut,
... indique une liaison en position $\alpha$ et ◀ une liaison en position $\beta$ tandis que une ligne ondulée (~~~) signifie que ces liaisons peuvent être en position $\alpha$ ou $\beta$,

avec un composé de formule générale V

$$[(R^6)_3P^{(+)}-(CH)_2)_4-COOR^4]X^{(-)} \qquad V$$

dans laquelle

$R^6$ signifie un alcoyle ayant I à 4 atomes de carbone ou phényle,
X Cl, Br ou I et
$R^4$ a la signification indiquée plus haut,

et en e qu'éventuellement on convertit un composé de formule III avec E = $-CH{=}CH-$ par hydrogénation en un composé de formule III avec E = $-CH_2-CH_2-$, et/ou on clive les groupes protecteurs présents en position $C_{11}$ et $C_{15}$ par solvolyse ou hydrogénolyse, et/ou en ce que pour la préparation des analogues de 13-thia-prostaglandine-E de formule générale III, on bloque en un stade quelconque sélectivement les groupes hydroxy en position $C_{11}$ et $C_{15}$ par des groupes protecteurs clivables par solvolyse ou hydrogénolyse, on oxyde le groupe hydroxy libre en position $C_9$ en groupe cétonique et l'on clive les groupes protecteurs en position $C_{11}$ et $C_{15}$ par solvolyse ou hydrogénolyse,
et/ou en e qu'éventuellement on convertit un composé de formule III avec $R^4$ = H, par réaction avec un agent d'estérification correspondant, en un autre composé de formule III avec $R^4$ = alcoyle ayant 1 à 4 atomes de carbone,
et/ou en e qu'éventuellement on convertit un composé de formule III avec $R^4$ = H, par réaction avec un composé de formule VI:

$$HO-Q-NH-COR^5 \qquad VI$$

en un autre composé de formule III avec $R^4 = -Q-NH-COR^5$,
et/ou en ce qu'éventuellement on convertit un composé de formule III avec $R^4$ = alcoyle ayant 1 à 4 atomes de carbone ou $-Q-NH-COR^5$, par réaction avec un agent de solvolyse, en un autre composé de formule III avec $R^4$ = H,
et/ou en ce qu'éventuellement on clive un composé de formule III en ses racémates et/ou énantiomères,
et/ou en ce qu'éventuellement on convertit un composé de formule III avec $R^4$ = H, par traitement avec une base, en un de ses sels physiologiquement inoffensifs ou le met en liberté, à partir d'un de ses sels, par traitement avec un acide.

8. Procédé selon la revendication 7, caractérisé en ce qu'on irradie avec de la lumière ultraviolette la 3$\alpha$-hydroxy-2$\beta$-(3-(3-chlorophénoxy)-2-hydroxypropylthio)-1$\beta$H,5$\beta$H-bicyclo(3.2.0)heptane-6-one, on fait réagir le 2-oxa-3-hydroxy-6$\beta$(3-(3-chlorophénoxy)-2-hydroxy-propylthio)-7$\alpha$-hydroxy-1$\beta$H,5$\beta$H-bicyclo(3.3.0)octane obtenu avec du bromure de carboxybutyltriphényl-phosphonium en obtenant ainsi l'acide 9$\alpha$,11$\alpha$,15-trihydroxy-16-(3-chlorophénoxy)-13-thia-17,18,19,20-tétranor-(Z)-5-prostènique.